# EUROPEAN PATENT APPLICATION

(11) **EP 4 806 434 A1**
(43) Date of publication of application: **16.09.2026**
(21) Application number: 25163568.6
(22) Date of filing: 13.03.2025
(51) Int. Cl.: A61K 31/426, A61K 9/00, A61K 47/40, A61P 31/22, A61K 31/522

(54) **PARENTERAL ADMINISTRATION FORMS OF ANTIVIRAL HELICASE-PRIMASE INHIBITORS**

(71) Applicant: Innovative Molecules GmbH, 81373 München (DE)
(72) Inventor: Kleymann, Gerald, 32107 Bad Salzuflen (DE); Gege, Christian, 89584 Ehingen (DE)
(74) Representative: Gille Hrabal Partnerschaftsgesellschaft mbB Patentanwälte

(57) **Abstract**

The invention relates to pharmaceutical compositions comprising a compound according to Formula (I) 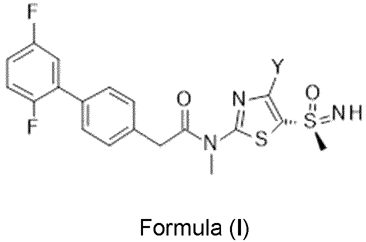 acting as a helicase-primase inhibitor, including its isotopic variants and pharmaceutically acceptable salts, co-crystals, hydrates and solvates thereof, and one or more cyclodextrins and the use thereof in the prophylaxis and treatment of herpes infections and mediated diseases.

## Description

### SUMMARY OF THE INVENTION

The present invention relates to pharmaceutical compositions comprising a compound according to Formula (**I**) 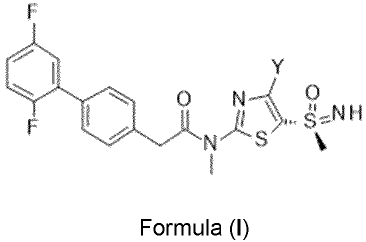 acting as a helicase-primase inhibitor, including its isotopic variants and pharmaceutically acceptable salts, co-crystals, hydrates and solvates thereof, and one or more cyclodextrins and the use thereof in the prophylaxis and treatment of herpes infections and mediated diseases. The composition is particularly suitable for parenteral administration of the compound of the Formula (I) to a human in therapeutically effective amounts yielding sufficient blood/plasma, neuronal tissue, nerves and meninges exposure.

### BACKGROUND AND PRIOR ART

The pandemic of herpes viral infections has plagued humanity since ancient times. Herpes is a lifelong infection with high incidence and prevalence causing herpes labialis, genital herpes or herpes keratitis and, less frequently, life-threatening herpes encephalitis, meningitis or disseminated disease involving hepatitis and pneumonia. Probably every human is infected with at least one strain of the human herpesviruses (HHV-1 to HHV-8) which poses a significant health burden worldwide. The prevalence of herpes simplex viruses (HSV-1) exceeds 60% in the general population, and HSV-2 infection rates are in the range of 15 to 30%. The alpha herpesviruses herpes simplex type 1 and 2 and varicella zoster virus (HHV-1 to HHV-3) are neurotrophic.

Disease symptoms often interfere with everyday activities and occasionally herpes simplex virus 1 and 2 (HSV-1 and HSV-2) infections are the cause of life-threatening encephalitis, viral meningitis (e.g. Mollaret's meningitis) or sight-impairing disease (keratitis), especially in neonates, elderly and the immunocompromised patient population such as transplant or cancer patients or patients with an inherited immunodeficiency syndrome or disease. After primary infection episomal HSV DNA persists for life in a latent state in nuclei of neurons of trigeminal or dorsal root sensory ganglia. Periodic reactivation occurs thereafter upon diverse stimuli, resulting in the shedding of contagious infectious virus and recurrent disease, often resulting in significant psychosocial distress for the patient. At least 30% of patients experience recurrences for life. Currently no cure is available.

The first nucleosidic drugs used in antiviral therapy, idoxuridine and trifluridine, were launched in the 1960s and 1980s for the topical treatment of herpes keratitis but posed safety concerns. Vidarabine was launched in the 1970s for systemic therapy of herpes encephalitis. The development of Acyclovir (ACV) can be seen as a milestone in antiviral therapy, although Acyclovir has moderate efficacy it offers a remarkable safety profile. Since its approval in 1981, systemic administration of ACV became the gold standard of herpes simplex and varicella zoster therapy. Valacyclovir (VACV) and famciclovir (FCV), prodrugs of ACV, and its congener Penciclovir (PCV) designed to improve systemic exposure after oral administration, were introduced in the 1990s and provide more convenient dosing of once to twice a day as compared to ACV, which requires administration three to five times a day.

So far, vaccines, interleukins, interferones, therapeutic proteins, antibodies, immunomodulators and small-molecule drugs with specific or non-specific modes of action lack either efficacy or the required safety profile to replace the nucleosidic drugs Acyclovir, Valacyclovir and Famciclovir as the first choice of treatment.

In case of herpes encephalitis or neonatal herpes high doses of ACV have to be administered to achieve sufficient exposure of the prodrug in the brain. However, neither oral nor intravenous formulations thereof have an impact on the neuronal latent viral reservoir.

At the dawn of the 21^{st} century, a novel class of antiviral drug compounds was developed, acting as helicase-primase inhibitors (HPIs), which is based on a different mechanism of action. However, to date, only the helicase-primase inhibitor Amenamevir is approved in Japan to treat herpes simplex and varicella zoster infections. Concerns about the use of Amenamevir as a treatment option in diseases involving the ganglia or central nervous system have been published and in the clinic, Amenamevir is not recommended for herpes infections of the central nervous system as summarized in Cureus 2022;16:e54775.

Further, there are currently aminothiazoles for the treatment of herpes infections under development (e.g. Pritelivir, HN0037, ABI-5366, ABI-1179), which are the most potent drugs in development today. These antiviral agents also act by inhibiting the herpesviral helicase-primase, display low resistance rates *in vitro* and showed superior efficacy in animal models compared to nucleosidic drugs. However, at least for Pritelivir, development is hampered by off-target carbonic anhydrase activity, reduced neuronal tissue and brain penetration and an unusual pharmacokinetic profile e.g. releasing an off-target, carbonic anhydrase active metabolite.

As mentioned above, alpha herpesviruses are neurotrophic viruses, which means that after infection they enter and settle in neuronal tissue leading to a persisting presence of these herpes viruses for life in neurons of the host in a latent form and a permanent neuronal exposure with the life-long risk of recurrent and periodically reactivating herpes infections. Such neuronal herpes virus exposure is further the cause of herpesvirus encephalitis (or herpes simplex encephalitis; HSE), which is thought to be caused by the transmission of herpes virus from a peripheral site on the face following HSV-1 reactivation or from neuronal tissue, along a nerve axon to the brain. The virus lies dormant in the ganglion of the trigeminal cranial nerve or in the neuronal tissue and gains access to the brain where it causes HSE.

The so far available therapy options are only moderately active reducing the course of the disease by 1 day in case of herpes labialis and genital herpes and reducing mortality of herpes encephalitis from 70 to 15% with high dose of intravenous Acyclovir. The pitfall for the current treatment options is that the therapy has no impact on the key feature of herpes simplex viruses, namely efficacy in reducing recurrent disease from the latent viral reservoir in ganglia of the nervous system that has been established for life during primary infection in the infected host.

In general, sufficient target exposure of the active drug compound is necessary for efficacy. To improve treatment and potentially attenuate chronically persistent HSV infections, exposure in neuronal tissues and brain is essential but is difficult to achieve with nucleoside analogs such as ACV or helicase primase inhibitors like Amenamevir or Pritelivir.

It is therefore important to provide highly active antiviral drugs with sufficient exposure in the neuronal system offering better efficacy when treating herpes encephalitis and having an impact on the reactivation competence of the neuronal latent viral reservoir, thereby allowing to treat and inactivate or eliminate also latent herpes viruses in neuronal tissue and nerves and avoid recurrence and reactivation of herpes infections or even the severe implications like HSE.

Known antiviral drugs as e.g. Amenamevir and the known aminothiazoles like Pritelivir have insufficient efficacy to enter neuronal tissue or to cross the blood-brain barrier to enter the brain and are therefore not able to provide an effective and eradicative cure for treating also latent or dormant forms of herpes viruses or even HSE.

The clinical Phase 1 results of the aminothiazole HN0037 and Pritelivir have been described e.g. in Clin. Pharmacol. Drug Develop. 2022;11:1467 and Clin. Pharmacol. Drug Develop. 2024;13:389, respectively.

WO2024/047508 outlines long-acting injectable pharmaceutical compositions, acting as subcutaneous or intramuscular depot formulations comprising helicase primase inhibitors like the therein described "Compound 1" 2-(3-(2',5'-difluoro-[1,1'-biphenyl]-4-yl)-2-oxotetra-hydropyrimidin-1(2*H*)-yl)-4-methylthiazole-5-sulfonamide (ABI-5366) or Pritelivir:

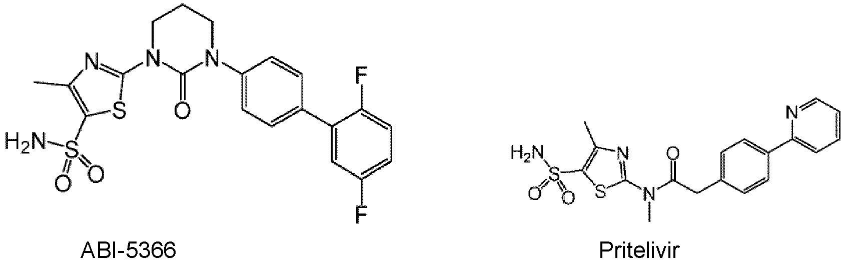

WO2024/224304 describes oral compositions of the above shown helicase-primase inhibitor "Compound 1" (ABI-5366).

Further helicase-primase inhibitor compounds based on indolinyl compounds are described in WO2023/225162.

WO2024049760 describes helicase-primase inhibitors with a cyclized urea moiety

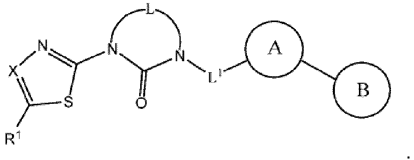

The helicase-primase inhibitor of the composition according to the present invention of the Formula (**I**) 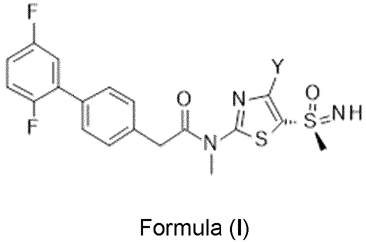 with Y being selected from CH₃ and CD₃ is also designated as **IM-250** and **d3-IM-250,** respectively ("d3-" indicating the deuteration). The international non-proprietary name (INN) of the compound IM-250 is Adibelivir.

This compound has first been described in WO2017/174640 describing the undeuterated (Y = CH₃) free base in its racemic form, while WO2019/068817 describes two enantiomers of the undeuterated (Y = CH₃) free base form, and in a general manner pharmaceutically acceptable salts thereof, respectively. WO2022/090409 describes deuterated analogs (Y = CD₃) of said compound and in a general manner pharmaceutically acceptable salts thereof. With respect to the characterization and preparation of deuterated forms of the compound of the Formula (**I**) referred to herein, as well as to the definition of deuteration, reference is made to said international application WO2022/090409, which in this respect is incorporated herein by reference.

Specific and selected salts and new solid crystalline salt forms and its deuterated analogues of the antiviral helicase-primase inhibitor compound according to the Formula (I) have been described in WO2023/135303.

Further, micronized solid crystalline forms of the hydrochloride salt of the compound according to Formula (I) have been described in WO2025/017032.

Further, some antiviral results of **IM-250** have been described in Sci. Transl. Med. 2021;13:eabf8668, Antivir. Res. 2021;195:105190 and Antivir. Res. 2023;219:105733.

Actually, a phase I clinical trial has been conducted to evaluate the general safety, tolerability, and pharmacokinetics of single doses of **IM-250** in healthy volunteers as described in https://clinicaltrials.gov/study/NCT06435507. According to the study plan disclosed therein, administration of **IM-250** in single doses of 50 mg, 100 mg, 200 mg and 400 mg are planned for administration to healthy volunteers to evaluate general safety and tolerability and **IM-250** plasma concentration for pharmacokinetic (PK) analysis.

Irrespective of said planned phase I study, the inventors of the present invention have found that oral administration of specific dose amounts and specific oral dose regimens comprising the oral administration of the active compound according to the Formula (I) or a pharmaceutically acceptable salt, co-crystal, hydrate or solvate thereof, are effective in the treatment of herpes viruses, and especially effective for the treatment of herpes simplex virus 1 and 2 (HSV-1 and HSV-2) as described in the European application EP24205645.5.

No specific galenic administration forms of compounds according to Formula (I) have been described so far and in view of the existing limitations of conventional herpes treatment options there is a continuing need to develop and provide improved dosage forms and administration regimes for highly effective, safe and stable administration. A main object of the invention is therefore the development of a pharmaceutical dosage form for the improved treatment of herpes infections (e.g. herpes simplex infections) and the associated severe implications and which allow to avoid the limitations and disadvantages of the so far described treatment options.

It is a particular object of the invention to provide highly active antiviral drug compositions with sufficient exposure of the active pharmaceutical ingredient (API) in the neuronal system offering better efficacy for treating also severe forms of herpes infections like herpes encephalitis and offering novel treatment options against the reactivation competence of the neuronal latent viral reservoir, allowing to treat and inactivate or eliminate also latent herpes viruses in neuronal tissue and nerves and avoid recurrence and reactivation of herpes infections or even the severe implications like HSE.

Further, a particular object of the invention can be seen in providing an administration form for helicase-primase inhibitors according to the Formula (I) being suitable for parenteral administration, particularly for intravenous administration and for infusion, as well as novel dosing and administration regimes to offer optimized and highly effective treatment of herpes infections.

In accordance with the above described objects of the invention a particular aim of the current invention is to provide a pharmaceutical composition which can conveniently be used in the medical practice, which allows to easily solubilize and deliver the drug compound according to Formula (**I**), or its isotopic variants, pharmaceutically acceptable salts, co-crystals, hydrates or solvates, parenterally (preferably via intravenous administration) at targeted concentrations for providing high clinical efficacy. Such compositions necessarily need to be safe and efficacious and should be stable in the relevant conditions and containers and should enable administration of an appropriate dose of the API, i.e. the compound according to Formula (**I**) or its isotopic variants, pharmaceutically acceptable salts, co-crystals, hydrates or solvates, over a reasonable timescale. In a further object, the composition should be able to be manufactured by a reliable and robust process for the preparation of parenteral dosage forms. Especially for providing infusion treatment options, a pharmaceutical composition easy to ship, store and handle should be provided, which allows the mixing of the parenteral (preferably intravenous) dose in an injection vial or infusion bag shortly before administration to the patient with standard equipment in a medical center or hospital. Finally, the composition should be stable under ambient conditions.

### DESCRIPTION OF THE INVENTION

The present invention relates to novel pharmaceutical compositions comprising the compound according to Formula (**I**) 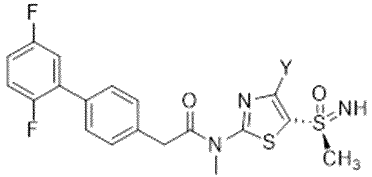 with Y being selected from CH₃ and CD₃, therewith covering the compound (**I**) and its deuterated analog, including its isotopic variants, pharmaceutically acceptable salts, co-crystals, hydrates and solvates, and one or more cyclodextrins, which allows to solve the objects described above.

The inventors of the present invention developed such pharmaceutical compositions as a novel dosage form, which was found particularly suitable for providing parenteral administration forms of the compound according to the Formula (I), including its isotopic variants, pharmaceutically acceptable salts, co-crystals, hydrates and solvates. Said novel pharmaceutical formulations can particularly be used to provide administration forms for intravenous administration or for infusion offering novel options for parenterally administering specific dose amounts and/or specific dose regimens as described herein in more detail. The novel pharmaceutical compositions and their parenteral, e.g. intravenous, administration allow a more effective treatment of herpes viruses, especially of herpes simplex virus 1 and 2 (HSV-1 and HSV-2) infections. The intravenous administration of the compound (I) in the specific dose strengths and/or specific dose regimens as described herein were found to be superior over so far described treatment approaches with conventional anti-herpes drugs like Acyclovir or Pritelivir.

The following Embodiments describe the present invention without being limited thereto:
[1] A pharmaceutical composition comprising a compound according to Formula (**I**) 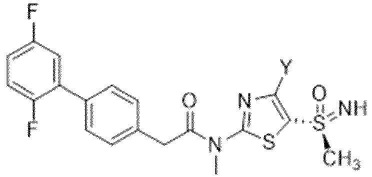
   wherein Y is selected from CH₃ and CD₃;
   or an isotopic variant, pharmaceutically acceptable salt, co-crystal, hydrate or solvate thereof,
   and one or more cyclodextrins.
[2] The pharmaceutical composition according to [1], which is a solid composition.
[3] The pharmaceutical composition according to [1] or [2], which is a lyophilizate, preferably a lyophilizate in a vial for dissolution in a pharmaceutically acceptable aqueous solution, preferably a physiological solution.
[4] The pharmaceutical composition according to [1], which further comprises a pharmaceutically acceptable aqueous solution, preferably a physiological solution, more preferably an intravenous injection solution or an infusion solution.
[5] The pharmaceutical composition according to [1] to [4] for the use in parenteral administration.
[6] The pharmaceutical composition according to [1] to [5] for the use in intravenous administration or for infusion.
[7] The pharmaceutical composition according to [1] to [6] comprising the compound of the Formula (I), or its isotopic variants, pharmaceutically acceptable salts, co-crystals, hydrates or solvates, in a dose strength of 10 to 600 mg per single dose;
   preferably in a dose strength of 10, 20, 25, 50, 100, 200, 400 or 600 mg per single dose;
   more preferably in a dose strength of 50, 100, 200 or 400 mg per single dose;
   even more preferably in a dose strength of 200 or 400 mg per single dose;
   calculated based on the weight of Formula (I) as the free base.
[8] The pharmaceutical composition according to [4] to [7], comprising the compound of the Formula (I), or its isotopic variants, pharmaceutically acceptable salts, co-crystals, hydrates or solvates, in a concentration of 0.5 to 10 mg/ml, preferably in a concentration of 0.8 or 2 mg/ml.
[9] The pharmaceutical composition according to [1] to [8] provided in a dosage form in the form of an ampule, vial, infusion bag, or mix-o-vial.
[10] The pharmaceutical composition according to [9], which is a ready-to-use formulation.
[11] The pharmaceutical composition according to [1] to [10], comprising a cyclodextrin selected from 2-hydroxypropyl-β-cyclodextrin (HP-β-cyclodextrin / HP-β-CD) and/or sodium sulfobutylether-β-cyclodextrin (SBE-β-cyclodextrin / SBE-β-CD), preferably comprising the cyclodextrin HP-β-cyclodextrin.
[12] The pharmaceutical composition according to [1] to [11], comprising the cyclodextrin HP-β-cyclodextrin and the molar ratio between the HP-β-cyclodextrin and the compound of the Formula (I) is at least 4:1.
[13] The pharmaceutical composition according to [1] to [12], comprising the cyclodextrin HP-β-cyclodextrin and the weight/weight ratio between the HP-β-cyclodextrin and the compound of the Formula (I) is at least 10:1, preferably about 15:1.
[14] The pharmaceutical composition according to [1] to [13], having a pH of 6.0 to 7.0, preferably pH 6.1 to 6.9, more preferably pH 6.2 to 6.8, more preferably pH 6.3 to 6.7, most preferred pH 6.5.
[15] The pharmaceutical composition according to [1] to [14], wherein the compound of the Formula (I) is selected from the group: 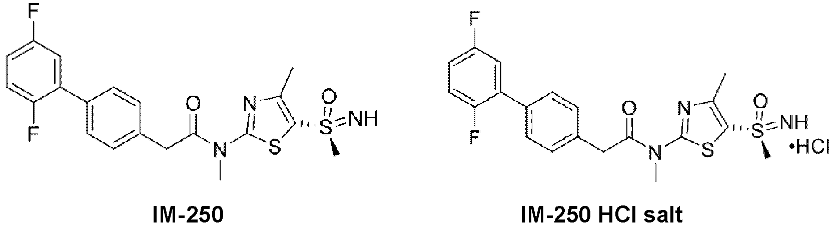 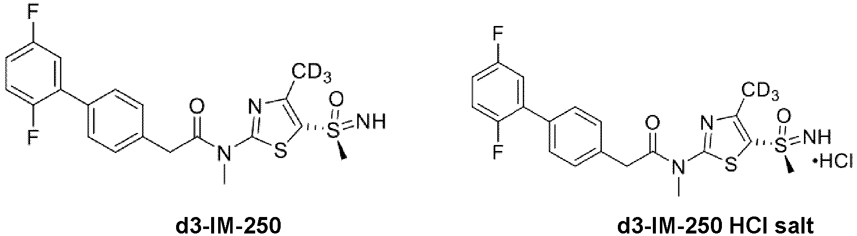 or a co-crystal, hydrate or solvate thereof.
[16] The pharmaceutical composition according to [1] to [15], wherein the compound of the Formula (**I**) is selected from crystalline forms of
   the **IM-250 HCl salt** characterized by an X-ray powder diffractogram comprising at least 4 of the following peaks (±0.2 degrees 2θ): 13.7, 17.0, 17.7, 19.8, 21.8 and 22.8;
      or
   the **d3-IM-250 HCl salt** characterized by an X-ray powder diffractogram comprising characteristic peaks (±0.2 degrees 2θ) at 9.3, 13.7 and 18.6;
   in each case as determined on a diffractometer using Cu-Kα radiation at a wavelength of 1.54 Å.
[17] The pharmaceutical composition according to [1] to [16], comprising the compound of the Formula (**I**) in the form of the **IM-250 HCl salt** 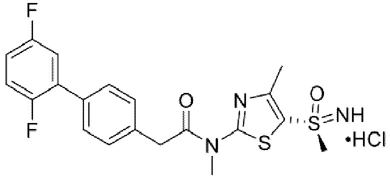 preferably in its crystalline form being characterized by an X-ray powder diffractogram comprising at least 4 of the following peaks (±0.2 degrees 2θ):13.7, 17.0, 17.7, 19.8, 21.8 and 22.8.
[18] The pharmaceutical composition according to [1] to [17], further comprising
   (i) one or more pharmaceutically acceptable carrier; and/or
   (ii) one or more excipients, such as a buffer and/or a tonicity adjusting agent, preferably a buffer selected from Na₂HPO₄ and a hydrate thereof, more preferably Na₂HPO₄•12 H₂O, and/or a tonicity adjusting agent selected from glucose and sodium chloride, more particularly sodium chloride; and/or
   (iii) one or more additional active substances being effective in treating a disease or disorder associated with viral infections (antiviral active compounds), preferably an additional active substance being effective in treating herpes infections, even more preferably Acyclovir.
[19] The pharmaceutical composition according to [4] to [18], comprising the compound of the Formula (I) in the form of the free base IM-250 or as its HCl salt 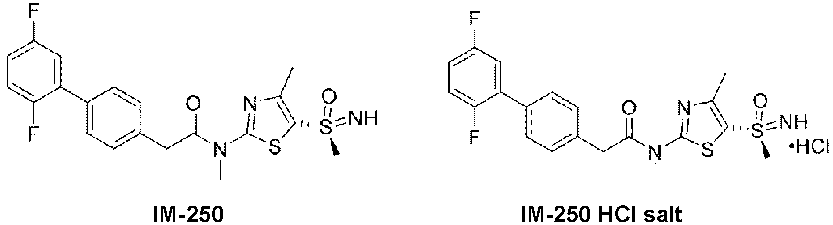
   a cyclodextrin selected from HP-β-cyclodextrin and/or SBE-β-cyclodextrin,
   a buffer, preferably Na₂HPO₄ or a hydrate thereof, more preferably Na₂HPO₄•12 H₂O, and
   a pharmaceutically acceptable aqueous solution, preferably a physiological solution.
[20] The pharmaceutical composition according to [1] to [19] in the form of a kit-of-parts comprising the pharmaceutical composition in lyophilized form, an injection vial, a cap, and optionally a pharmaceutically acceptable aqueous solution, preferably a physiological solution for dissolution of the lyophilizate in the vial, and optionally infusion equipment, including a syringe, a needle, a butterfly, winged infusion set etc.
[21] The pharmaceutical composition according to [1] to [20] for the use in the prophylaxis and treatment of a disease or disorder associated with viral infections caused by herpes viruses, such as in particular by herpes simplex viruses.
[22] The pharmaceutical composition according to [1] to [20] for the use in treating and reducing or eliminating latent (dormant) forms of herpes viruses in neuronal tissue, nerves and meninges, preferably for avoiding or preventing recurrence and reactivation of herpes infections or even severe implications associated therewith, such as Mollaret's meningitis or herpes simplex encephalitis (HSE).
[23] The pharmaceutical composition according to [1] to [20] for the use in the prophylaxis and treatment of herpes infections, in particular Herpes simplex infections, in patients displaying Herpes labialis, Herpes genitalis and Herpes-related keratitis, Herpetic esophagitis, Herpetic pneumonia, Alzheimer's disease, encephalitis, Mollaret's meningitis, pneumonia, hepatitis; in patients with a suppressed immune system, such as AIDS patients, cancer patients, patients having a genetic immunodeficiency, transplant patients; in new-born children and infants; in Herpes-positive patients, in particular Herpes-simplex-positive patients, for suppressing recurrence or viral shedding (suppression therapy); or in patients, in particular in Herpes-positive patients, in particular Herpes-simplex-positive patients, who are resistant to nucleosidic antiviral therapy such as Acyclovir, Penciclovir, Famciclovir, Ganciclovir, Valacyclovir and/or compounds such as the phosphonates Foscarnet or Cidofovir.
[24] The pharmaceutical composition according to [1] to [20] for the use in the prophylaxis and treatment of neurodegenerative diseases caused by viruses, including Alzheimer's disease caused by viruses, such as caused by Herpes simplex viruses.
[25] The pharmaceutical composition according to [1] to [20] for the use in the prophylaxis and treatment of diseases caused by or associated with Herpes simplex infections, which are selected from encephalitis, neonatal herpes esophagitis and Alzheimer's disease.
[26] The pharmaceutical composition for the use according to [21] to [25], comprising the parenteral administration of the composition with a daily dose amount of 10 to 400 mg of the compound of Formula (**I**), or its isotopic variants, pharmaceutically acceptable salts, co-crystals, hydrates or solvates;
   preferably with a daily dose amount of 10, 20, 25, 50, 100, 200 or 400 mg;
   more preferably with a daily dose amount of 50, 100, 200 or 400 mg;
   even more preferably with a daily dose amount of 200 or 400 mg;
   calculated based on the weight of the compound of Formula (I) as the free base.
[27] The pharmaceutical composition for the use according to [26], wherein the daily dose amounts are administered by single daily doses.
[28] The pharmaceutical composition for the use according to [21] to [27], comprising the administration of the composition intravenously or via infusion.
**[29]** The pharmaceutical composition for the use according to [21] to [28], comprising intermittent administration of the composition with same or different daily doses of the compound of Formula (**I**), or its isotopic variants, pharmaceutically acceptable salts, co-crystals, hydrates or solvates, in repeating equal or varying intervals of between 4 to 21 days.
[30] The pharmaceutical composition for the use according to [29], comprising administration of the composition over a total treatment period of between 14 to 21 days with administration of the compound with a dose strength of 200-600 mg as a single initial loading dose on the first administration day, followed by administration of the composition with a maintenance dose strength of 25-100 mg per daily dose on the following days.
[31] The pharmaceutical composition for the use according to [29] or [30] in a suppression therapy for the treatment or elimination of latent forms of herpes viruses in neuronal tissue and nerves and/or for the prevention and treatment of recurrence and reactivation of herpes infections or severe implications associated therewith, wherein the intermittent parenteral administration is preferably used for treating and/or curing chronic herpes infections.
[32] The pharmaceutical composition for the use according to [21] to [28], wherein the daily dose amounts are administered once only per treatment of an episode.
[33] The pharmaceutical composition for the use according to [21] to [32], comprising the parenteral administration of the composition to achieve a target minimum concentration of the compound of the Formula (I)
   - in the spinal cord of ≥ 1.0 µM, preferably ≥ 5.0 µM, more preferably ≥ 7.0 µM, more preferably ≥ 9.0 µM, even more preferably ≥ 10 µM; and/or
   - in the brain of ≥ 500 nM, preferably ≥ 1.0 µM, more preferably ≥ 5.0 µM, more preferably ≥ 7.0 µM, even more preferably ≥ 9.0 µM; and/or
   - in the trigeminal ganglia of ≥ 1.0 µM, preferably ≥ 5.0 µM, more preferably ≥ 7.0 µM, more preferably ≥ 9.0 µM, even more preferably ≥ 10 µM; and/or
   - in the sacral ganglia of ≥ 2.0 µM, preferably ≥ 5.0 µM, more preferably ≥ 7.0 µM, even more preferably ≥ 9.0 µM; and/or
   - in the cerebrospinal fluid (CSF) of ≥ 0.05 µM, preferably ≥ 0.07 µM, more preferably ≥ 0.09 µM.
[34] The pharmaceutical composition for the use according to [21] to [33], comprising the parenteral administration of the composition
   - to achieve a target minimum concentration in plasma or blood, preferably plasma, of at least 500 nM, preferably at least 1 µM and more preferably at least 2 µM; and/or
   - to maintain a target minimum plasma concentration of at least 500 nM for at least 6 hours, preferably to maintain a target minimum plasma concentration of at least 1 µM for at least 24 hours, preferably for at least 5 days, more preferably for at least 8 days, more preferably for at least 10 days, more preferably for at least 14 days; and/or
   - to maintain the target minimum plasma concentration for at least 24 hours up to 5 days, preferably for at least 24 hours up to 8 days, more preferably for at least 24 hours up to 10 days, more preferably for at least 24 hours up to 14 days; and/or
   - to achieve an essentially steady stable plasma or blood, preferably plasma, concentration level of the compound of Formula (**I**) around about 500 nM, preferably about 1 µM and more preferably about 2 µM; and/or
   - to achieve an apparent clearance rate (CL/F) of equal to or less than about 2.5 L/h, preferably equal to or less than 1 L/h more preferably less than 0.75 L/h.
[35] The pharmaceutical composition for the use according to [32], wherein a single dose in a once-only treatment per episode is parenterally administered in an amount to achieve a target minimum concentration as defined in [33] or [34].
[36] The pharmaceutical composition for the use according to [21] to [35], wherein the treatment by administering the composition to the subject to be treated is started at between 0 to 4 days post infection or recurrence, preferably recurrence.
[37] The pharmaceutical composition according to [1] to [20] for the use in a combination therapy for the prophylaxis or treatment as defined in [21] to [36], wherein the combination therapy comprises co-administration of the compound of Formula (**I**), or its isotopic variants, pharmaceutically acceptable salts, co-crystals, hydrates or solvates, with one or more additional pharmaceutically active compounds, preferably one or more antiviral compounds, more preferably Acyclovir,
   wherein the co-administration of the combination therapy is carried out in a fixed dose combination therapy by co-administration of the compound of the Formula (I) and the one or more additional pharmaceutically active compounds in a fixed-dose formulation or
   wherein the co-administration of the combination therapy is carried out in a free dose combination therapy by co-administration of the compound of the Formula (I) and the one or more additional pharmaceutically active compounds in free doses of the respective compounds, either by simultaneous administration of the individual compounds or by sequential administration of the individual compounds over a time period.
[38] A process for the preparation of the pharmaceutical composition according to [1] to [20], comprising the following steps:
   (a) preparing a solution of cyclodextrin in water to obtain an aqueous cyclodextrin-solution and optionally adjusting the pH to an acidic pH of < 5.0, preferably pH ≤ 4.0, more preferably pH ≤ 3.0, even more preferably pH ≤ 2.0,
   (b) adding and solubilizing the compound of the Formula (I), or its isotopic variant, pharmaceutically acceptable salt, co-crystal, hydrate or solvate, in the cyclodextrin-solution of step (a) so that the resulting solution has an acidic pH of < 5.0, preferably pH ≤ 4.0, more preferably pH ≤ 3.0, even more preferably pH ≤ 2.0,
   (c) subsequently adding a buffer to the solution of step (b) and increasing the pH of the solution to a pH of 6.0 to 7.0, preferably 6.1 to 6.9, more preferably 6.2 to 6.8, more preferably 6.3 to 6.7, most preferred 6.5,
   (d) freeze-drying of the solution of step (c) to obtain a lyophilizate.
[39] The process according to [38], wherein in step (b) a HCl salt of the compound of the Formula (I) is added.
[40] The process according to [38], wherein in step (b) the free base of the compound of the Formula (I) is added after adjustment of the pH of the cyclodextrin-solution of step (a) to acidic pH of < 5.0, preferably pH ≤ 4.0, more preferably pH ≤ 3.0, even more preferably pH ≤ 2.0, preferably by addition of an acid, more preferably hydrochloric acid (HCl).
[41] The process according to [38] to [40], wherein the buffer added in step (c) is Na₂HPO₄ or a hydrate thereof, particularly Na₂HPO₄•12 H₂O.
[42] The process according to [38] to [41], further comprising the following step:
   (e) adding a pharmaceutically acceptable aqueous solution, preferably a physiological solution, more preferably an intravenous injection solution or an infusion solution to the lyophilizate of step (d) to obtain an injectable parenteral pharmaceutical composition.
[43] The process according to [42], wherein in step (e) the pharmaceutically acceptable aqueous solution, preferably the physiological solution, more preferably the intravenous injection solution or infusion solution, is added to the lyophilizate of step (d) in an amount to dilute the lyophilizate to obtain the target concentration of the compound of the Formula (I) in the injectable parenteral pharmaceutical composition.
[44] The process according to [38] to [43], wherein in one or more of the steps (a) to (c) and (e) one or more pharmaceutically acceptable carrier and/or one or more excipients, and/or one or more additional active substances being effective in treating a disease or disorder associated with viral infections (antiviral active compounds) are added.
[45] The process according to [44], wherein additional excipients are selected from buffer, preferably Na₂HPO₄ and hydrates thereof, more preferably Na₂HPO₄•12 H₂O, and from tonicity adjusting agents, preferably selected from glucose and sodium chloride, more preferably sodium chloride, and additional active substances being effective in treating herpes infections are selected from antiviral drug compounds, preferably Acyclovir.
[46] The process according to [38] to [45], wherein the compound of the Formula (**I**), or its isotopic variants, pharmaceutically acceptable salt, co-crystal, hydrate or solvate, added in step (b) is micronized, preferably micronized having with a d₉₀ value of less than or equal to about 20.0 µm, more preferably micronized with a d₉₀ value of less than or equal to about 10.0 µm.

In summary, the present invention relates to novel pharmaceutical compositions and dosage forms for administering the compound of the Formula (I) as defined herein.

### General Definitions

Generally, when used herein, references to "about" a value or parameter herein includes (and describes) embodiments that are directed to that value or parameter *per se.* For example, description referring to "about x" includes description of "x". Also, the singular forms "a" and "the" include plural references unless the context clearly dictates otherwise. Thus, e.g. reference to "the compound" includes a plurality of such compounds and reference to "the salt" includes reference to one or more salts and equivalents thereof known to those skilled in the art.

The term "substantially" when referring, for example, to an XRPD pattern, a value or range, includes a pattern, value or range that is not necessarily identical to those depicted herein, but that falls within the limits of experimental error or deviations when considered by one of ordinary skill in the art and being as accurate as the method used to measure it and as the technical tolerance of the measurement method allows.

Unless the context requires otherwise, throughout the present specification and claims, the word "comprise" and variations thereof, such as, "comprises" and "comprising" are to be construed in an open, inclusive sense, that is as "including, but not limited to".

### Drug Compound and Pharmaceutical Composition

In a first aspect, the invention relates to pharmaceutical compositions comprising a compound according to Formula (I) 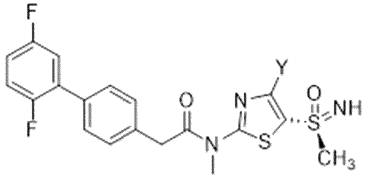
wherein Y is selected from CH₃ and CD₃;
or an isotopic variant, pharmaceutically acceptable salt, co-crystal, hydrate or solvate thereof, and one or more cyclodextrins.

A "pharmaceutical composition" or "pharmaceutical dosage form" as referred to herein relates to a pharmaceutically acceptable formulation of the compound of Formula (**I**), including its pharmaceutically acceptable salts, co-crystals, hydrates and solvates, with at least one pharmaceutically acceptable excipient or auxiliary component allowing to formulate the compound of the Formula (I) for the intended dosage form and administration route.

The term "pharmaceutical composition" mainly describes the galenic formulation of the compound of the Formula (I), i.e. its composition with excipients, auxiliaries etc.. The term "pharmaceutical dosage form" further describes the dosage form and how the pharmaceutical formulation is provided in the market. For example, a parenteral pharmaceutical composition according to the invention can be present in solid or liquid form comprising the components as described in more detail below. Such solid or liquid parenteral pharmaceutical composition can be provided in a pharmaceutical dosage form in a vial, ampule, etc. If the pharmaceutical composition is provided in a dosage form in solid form, for the parenteral administration said dosage form necessarily needs to be solubilized prior to administration to the patient. If the pharmaceutical composition is provided in a dosage form in liquid form, it can be administered directly or needs to be further diluted (e.g. if provided as a concentrate). Accordingly, a pharmaceutical dosage form for parenteral administration according to the invention may cover ready-to-use formulations as well as dosage forms which need further preparation steps prior to administration to the patient. In this respect, the pharmaceutical dosage forms and their handling for parenteral administration as referred to herein comply with conventional techniques and practice and are well-known to the practitioner.

"Pharmaceutically acceptable" or "physiologically acceptable" refer to compounds, salts, compositions, dosage forms and other materials which are useful in preparing a pharmaceutical dosage form that is suitable for human pharmaceutical use.

Even if not explicitly referred to, throughout this specification references to the compound of Formula (**I**) include the crystalline forms, salts, co-crystals, hydrates and/or solvates of the compounds depicted in Formula (**I**), unless explicitly indicated otherwise or apparent from the context.

### Deuterated Analogs

In accordance with the definition of the Formula (I), the drug compounds comprised in the novel pharmaceutical compositions can also be used in the form of deuterated analogs and isotopic variants.

As defined in the international application WO2022/090409 "deuteration", "deuterium labelled", "deuterium substituted" or "deuterated" means to replace hydrogen atom(s) of the compound of Formula (**I**) by deuterium (²H, represented by "D").

In deuterated analogs of the compounds of Formula (**I**), any hydrogen can be replaced by deuterium, however preferably the residue Y represents CD₃.
As described in the international application WO2022/090409 such deuterated aminothiazole compounds were found to exhibit increased resistance to metabolism and were thus considered as useful for increasing the half-life of the compound of Formula (**I**), compared to a respective undeuterated compound, when administered to a mammal, e.g. a human. The concentration of deuterium may be defined by an isotopic enrichment factor. In the compounds of this disclosure any atom specifically designated as a deuterium (D) is meant to represent deuterium with an isotopic purity of at least 50%, preferably an isotopic purity of at least 95%, more preferably an isotopic purity of at least 99%. The percentage of deuterium incorporation can be obtained by quantitative analysis using a number of conventional methods, such as mass spectroscopy (peak area) or by quantifying the remaining residual ¹H-NMR signals of the specific deuteration site compared to signals from internal standards or other, non-deuterated ¹H signals in the compound. It will be recognized that some variation of natural isotopic abundance occurs in a synthesized compound depending upon the origin of chemical materials used in the synthesis. Thus, preparation of non-deuterated analogs of compounds of the present invention will inherently contain small amounts of deuterated isotopologues. The concentration of naturally abundant stable hydrogen and carbon isotopes, notwithstanding this variation, is small and immaterial as compared to the degree of stable isotopic substitution of compounds of this invention. The term "isotopic enrichment factor" at a particular position normally occupied by hydrogen refers to the ratio between the abundance of deuterium at the position and the natural abundance of deuterium at that position. By way of example, an isotopic enrichment factor of 3500 means that the amount of deuterium at the particular position is 3500-fold the natural abundance of deuterium, or that 52.5% of the compounds have deuterium at the particular position (i.e., 52.5% deuterium incorporation at the given position). The abundance of deuterium in the oceans of Earth is approximately one atom in 6500 hydrogen atoms (about 154 parts per million (ppm)). Deuterium thus accounts for approximately 0.015 percent (on a weight basis, 0.030 percent) of all naturally occurring hydrogen atoms in the oceans on Earth; the abundance changes slightly from one kind of natural water to another.

The deuterated compounds of this disclosure are preferably characterized by an isotopic enrichment factor of at least 6300, or by a deuteration degree of at least 95%. More preferably by an isotopic enrichment factor of at least 6500, or by a deuteration degree of at least 98%.

In addition to deuterated compounds, the compounds as defined by the Formula (I) also cover other "isotope variants", i.e. compounds which may contain one or more other isotopically labeled atoms. Examples of additional isotopes besides deuterium that can be incorporated into compounds of the disclosure include further isotopes of hydrogen (i.e. tritium or ³H), as well as isotopes of carbon, nitrogen, oxygen, phosphorous, fluorine and chlorine, such as, but not limited to ¹¹C, ¹³C, ¹⁴C, ¹⁵N, ¹⁸F, ¹⁹F, ³¹P, ³²P, ³⁵P, ³⁵S, ³⁶Cl and ¹²⁵I. The disclosure further comprises various isotopically labelled compounds into which radioactive isotopes such as ³H, ¹³C and ¹⁴C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays or radioactive treatment of patients.

### Salts and Solvates

If in the pharmaceutical compositions, pharmaceutical parenteral dosage forms and/or the treatment methods of the present invention the compound of the Formula (**I**) is used in the form of a pharmaceutically acceptable salt, such salt is preferably a HCl salt and the deuterated forms thereof: 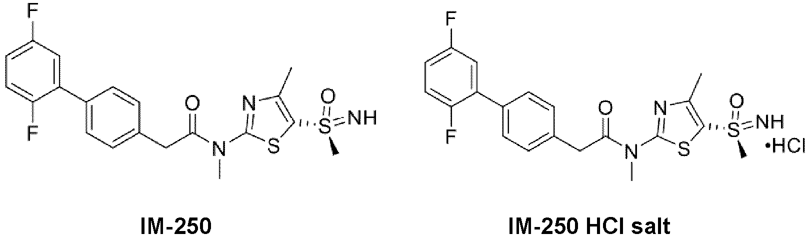 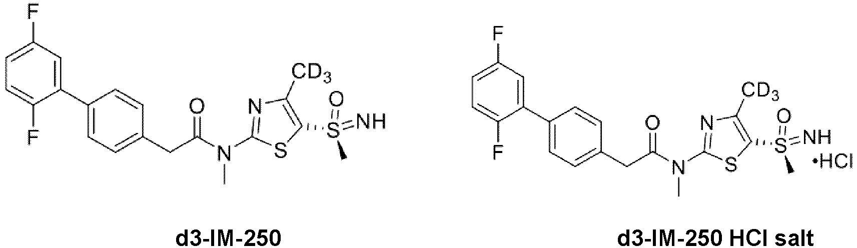 or a co-crystal, hydrate or solvate thereof.

Among such selected pharmaceutically acceptable salts it is particularly preferred to use a salt having a crystalline form characterized by an X-ray powder diffractogram comprising at least 4 of the following peaks (±0.2 degrees 2θ):

| | |
|---|---|
| **IM-250 HCl salt:** | 13.7, 17.0, 17.7, 19.8, 21.8 and 22.8, |

or a crystalline form with an X-ray powder diffractogram comprising characteristic peaks (±0.2 degrees 2θ) at

| | |
|---|---|
| **d3-IM-250 HCl salt:** | 9.3, 13.7 and 18.6 |

in each case as determined on a diffractometer using Cu-Kα radiation at a wavelength of 1.54 Å. With respect to the characterization and preparation of such crystalline forms of the compounds of the Formula (**I**) reference is made to the international application WO2023/135303, which is incorporated herein by reference.

In a particularly preferred aspect of the present invention the compound of the Formula (I) for the use as defined herein is present in the form of its HCl salt, wherein hydrochloride and (*S*)-2-(2',5'-difluoro-[1,1'-biphenyl]-4-yl)-*N*-methyl-*N*-(4-methyl-5-(*S*-methylsulfonimidoyl) thiazol-2-yl)acetamide (**IM-250**) or its deuterated form (**d3-IM-250**), respectively, are present in a 1:1 molar ratio.

The non-deuterated form of the compound of Formula (**I**) (**IM-250**) and its HCl salt are preferred, most preferred is the HCl salt of the non-deuterated form (**IM-250 HCl salt**).

The compounds of the present disclosure may be present in the form of solvates, such as those which include as solvate water, or pharmaceutically acceptable solvates, such as alcohols, in particular ethanol. A "solvate" is formed by the interaction of a solvent and a compound. When the solvent is water, the "solvate" is a "hydrate". It is understood, that also a salt of the present disclosure can include a solvate.

Suitable solvents for salt- and solvent formation of the compounds according to Formula (**I**) as defined herein comprise: sulfoxides (e.g. dimethylsulfoxid (DMSO)), amides (e.g. N,N-dimethylformaide (DMF), N-methyl-2-pyrrolidone (NMP)), nitriles, e.g. acetonitrile, dichloromethane (DCM), alcohols, such as especially methanol, ethanol, 2-propanol (isopropanol), aldehydes, ketones, especially acetone, ethers, e.g. tetrahydrofuran (THF) or dioxane, esters, e.g. ethyl acetate, or alkanes, such as especially pentane, hexane, heptane or cyclohexane and water, and mixtures thereof.

In principle, it is also possible to formulate other helicase-primase inhibitor compounds with cyclodextrins to provide dosage forms as described herein for parenteral administration, e.g. intravenously or via infusion, for treating viral infections as described herein. Such other helicase-primase inhibitors may be selected from those described in the above cited prior art, including Amenamevir, aminothiazoles like Pritelivir, HN0037, ABI-5366 (Compound 1 according to WO2024/047508), ABI-1179, or indolinyl compounds as described in WO2023/225162 (including the therein described Example Compounds No. 1 to 20, 22 to 29, 31, 33 to 41, 43 to 53, 56 to 65, 68 to 83, 85 to 119, 123, and 125 to 141), and helicase-primase inhibitors with a cyclized urea moiety (according to WO2024049760) including the therein described Example Compounds No. 5, 104, 259 and 314. However, in view of the herein discussed advantages and benefits of the compounds according to Formula (I) of the present invention, such alternative helicase-primase inhibitor compounds known from the prior art are not preferred.

### Cyclodextrins

The compound according to Formula (**I**) has limited aqueous solubility across physiologically relevant pHs (kinetic solubility of <0.1 mg/mL for the free base in phosphate-buffered saline at pH 7.4). In order to enable a fast, safe and effective administration of the compound according to Formula (**I**) or a pharmaceutically acceptable salt, co-crystal, hydrate or solvate thereof, and to elicit the required therapeutic effects, the compound according to Formula (**I**) needs to be solubilized at higher concentration than its aqueous solubility allows.

Particularly for parenterally administrable drug compounds solubilization is crucial and finding a suitable way to solubilize the compounds in a safe, reliable, stable and pharmaceutically acceptable manner in high amounts poses a challenge to the galenic development.

Generally, different ways to solubilize poorly soluble compounds for pharmaceutical administration forms exist. Typical approaches include the use of pharmaceutically acceptable solvents and co-solvents or emulsifier, like PEG300, PEG400, Lipofundin, Capmul, polysorbate, propylene glycol or ethanol or the use of surfactants like Tween^{®} 80 or Kolliphor^{™} ELP may be considered. Further approaches aim at controlling the pH of a drug compound.

The suitability of a solubilization measure strongly depends on the drug compound with its specific structure, physical and physico-chemical properties, the desired administration form, the targeted drug concentration and administration route, the release and pharmacokinetic properties as well as the intended treatment and indication field. Depending on these specific circumstances the suitable solubilization approach is not easily foreseeable but requires intensive galenic development work.

WO2024/047508 describes to formulate the helicase primase inhibitors Pritelivir and "Compound 1" 2-(3-(2',5'-difluoro-[1,1'-biphenyl]-4-yl)-2-oxotetrahydropyrimidin-1(2*H*)-yl)-4-methylthiazole-5-sulfonamide (ABI-5366) as an aqueous based microsuspension depot composition to provide long-acting injectable administration forms for subcutaneous or intramuscular administration. It is described therein to solubilize the helicase-primase inhibitors Pritelivir and "Compound 1" in biocompatible organic solvents like N-methyl-2-pyrrolidone (NMP), dimethylacetamide (DMA), dimethyl sulfoxide (DMSO), benzyl alcohol, benzyl benzoate and mixtures thereof, wherein such organic solvent based solutions may further comprise an aqueous media such as aqueous buffer or saline or a surfactant such as a polyethylene glycol fatty ester, e.g. PEG-15 hydroxystearate.

The inventors of the invention found that the compound of the Formula (I) surprisingly offers the possibility to be administered parenterally, i.e. intravenously, in significantly lower doses than conventional herpes simplex drugs like Acyclovir or Pritelivir due to its higher efficacy and the potential to maintain a stable and constant high plasma level over a much longer time period. It has further surprisingly been found that the compounds according to the Formula (I) have significant efficacy to enter neuronal tissue or to cross the blood-brain barrier to enter the brain and are therefore particularly suitable to provide an effective and eradicative cure for treating also latent or dormant forms of herpes viruses or even HSE. Based on these surprising findings the inventors of the present invention developed novel pharmaceutical compositions for the helicase-primase inhibitor compound of the Formula (I) for parenteral, particularly intravenous, administration based on cyclodextrins as described herein.

The cyclodextrins of the pharmaceutical compositions described herein can be a natural or derived cyclodextrin. Natural cyclodextrins comprise three well-known industrially produced (major and minor) cyclic oligosaccharides. The most common natural cyclodextrins are α, β, and γ consisting of 6, 7, and 8 glucopyranose units. Derived cyclodextrins include hydroxyalkylated cyclodextrins selected from the group consisting of hydroxyethyl cyclodextrin, hydroxypropyl cyclodextrin and hydroxybutyl cyclodextrin. In a particular embodiment, the cyclodextrin is the β-cyclodextrin itself or its derivatives. The derivatives herein mean β-cyclodextrins having various substituents, including methyl-β-cyclodextrin, ethyl-β-cyclodextrin, (2-hydroxypropyl)-β-cyclodextrin, (3-hydroxypropyl)-β-cyclodextrin, (2-hydroxyethyl)-β-cyclodextrin, carboxymethyl-β-cyclodextrin, carboxyethyl-β-cyclodextrin, succinyl-β-cyclodextrin carboxymethyl-ethyl-β-cyclodextrin, diethyl-β-cyclodextrin, dimethyl-β-cyclodextrin, trimethyl-β-cyclodextrin, glucosyl-β-cyclodextrin, hydroxybutenyl-β-cyclodextrin, β-cyclodextrin phosphate, β-cyclodextrin sulfate, maltosyl-β-cyclodextrin, randomly methylated-β-cyclodextrin, sulfobutylether-β-cyclodextrin, 2-selenium-bridged β-cyclodextrin, and 2-tellurium-bridged β-cyclodextrin. Besides β-cyclodextrin, 2-hydroxypropyl-γ-cyclodextrin can be used in the present invention. Derived cyclodextrins also include polymerized cyclodextrins, which are high molecular weight compounds, either water-soluble or insoluble. Examples of polymerized cyclodextrins comprise soluble anionic β-cyclodextrin polymer, soluble γ-cyclodextrin polymer and epichlorohydrin β-cyclodextrin polymer.

It is possible to use one cyclodextrin or mixtures of selected cyclodextrins.

A preferred cyclodextrin is "2-hydroxypropyl-β-cyclodextrin", which is also named "hydroxypropyl-β-cyclodextrin" or "HP-β-cyclodextrin" or "hydroxypropylbetadex", often abbreviated with "HP-β-CD". In particular, HP-β-CD is marketed with the following product names: Cavitron^{™} W7HP7 (typical degree of substitution: 6.0-8.0; approximate molecular weight: 1520), Cavitron^{™} W7HP5 (typical degree of substitution: 4.1-5.1; approximate molecular weight: 1410), Cavasol^{®} W7 HP, Kleptose^{™} HPB or Kleptose^{™} HP.

Another preferred cyclodextrin is "SBE-β-cyclodextrin", which is also named "sodium sulfobutylether-β-cyclodextrin" or "betadex sulfobutyl ether sodium", often abbreviated with "SBE-β-CD". In particular, the SBE-β-CD is marketed with the following product names: Dexsolve^{™} or Captisol^{™}.

In the pharmaceutical composition of the invention one or more cyclodextrins selected from those described above can be used.

In a preferred aspect of the invention the pharmaceutical composition comprises a cyclodextrin selected from 2-hydroxypropyl-β-cyclodextrin (HP-β-cyclodextrin / HP-β-CD) and sodium sulfobutylether-β-cyclodextrin (SBE-β-cyclodextrin / SBE-β-CD) or a mixture thereof, preferably the compositions comprise the cyclodextrin HP-β-cyclodextrin. It is even more preferred that the composition comprises HP-β-cyclodextrin or SBE-β-cyclodextrin as the sole cyclodextrin, even more preferably the composition comprises HP-β-cyclodextrin as the sole cyclodextrin.

The molar ratio between the cyclodextrin and the compound of the Formula (I) is preferably at least 2:1, more preferably at least 3:1 and most preferably ≥ 4:1.

In embodiments of the invention, wherein the preferred cyclodextrin HP-β-cyclodextrin is selected, it is further preferred that the molar ratio between the HP-β-cyclodextrin and the compound of the Formula (I) is at least 3:1, preferably at least 4:1.

In embodiments of the invention, wherein the preferred cyclodextrin SBE-β-cyclodextrin is selected, it is further preferred that the molar ratio between the SBE-β-cyclodextrin and the compound of the Formula (I) is at least 2:1 or 3:1.

In embodiments of the invention, wherein the preferred cyclodextrin HP-β-cyclodextrin is selected, it is further preferred that the weight/weight ratio between the HP-β-cyclodextrin and the compound of the Formula (I) is at least 10:1, preferably about 15:1.

### Further Excipients

Depending on the concrete dosage form the pharmaceutical composition according to the invention can further comprise additional pharmaceutically acceptable auxiliaries, excipients, carrier, solvents etc., which can be selected from any of the usual pharmaceutical media that may be employed in parenteral dosage forms, such as, for example, water, glycols, oils, alcohols, flavoring agents, preservatives, coloring agents and the like.

Preferred examples of additional excipients comprise buffer and tonicity adjusting agents.

A "buffer" can be used to prevent changes in the pH of a solution and for controlling the pH during the manufacturing process. For parenteral administration forms a physiological pH of the composition is preferred. The pharmaceutical compositions of the present invention preferably have a pH of 6.0 to 7.0, preferably pH 6.1 to 6.9, more preferably pH 6.2 to 6.8, more preferably pH 6.3 to 6.7, most preferred about pH 6.5. Such pH of the composition can be obtained by adding a pH adjusting agent or suitable buffer. Examples of suitable buffer are well-known to the skilled formulator. A preferred buffer used in the pharmaceutical compositions of the invention and for the preparation thereof are Na₂HPO₄ and its hydrates, like more preferably Na₂HPO₄•12 H₂O.

Adjusting the pH of the pharmaceutical composition in the above range between 6.0 to 7.0 further stabilizes the drug compound (API) in the formulation and prevents its cleavage.

It is further possible to add a tonicity adjusting agent. "Tonicity adjusting agent" means a pharmaceutically acceptable compound which can be added to a formulation to make it isotonic with human plasma. Tonicity adjusting agents include for example dextrose, glucose, mannitol, sucrose, lactose, trehalose, glycerin and sodium chloride, in particular a 0.9% isotonic solution of sodium chloride. Tonicity is the "effective osmolality" and is equal to the sum of the concentrations of the solutes which have the capacity to exert an osmotic force across the membrane. Parenteral formulations, preferably for intravenous administration, should be isotonic with blood plasma. Preferred tonicity adjusting agents according to the invention are selected from glucose and sodium chloride, more particularly sodium chloride.

### Drug Doses and Administration Regime

The pharmaceutical composition of the present invention comprises an effective amount of the compounds of the Formula (I) as defined herein.

The term "effective amount" is meant to include the amount of the active compound that, when administered, is sufficient to prevent development of, or alleviate to some extent, an infection or one or more of the symptoms of the disorder, disease, or condition being treated. The term "effective amount" also refers to the amount of the active compound that is sufficient to elicit the biological or medical response of a cell, tissue, system, animal, or human, which is being sought by a researcher, veterinarian, medical doctor, or clinician. In the particular aspects of the present invention, "effective amount" is meant to include the amounts being able to achieve the defined and desired target levels defined herein.

Compounds may, in some embodiments, be administered to a subject (including a human) who is at risk or has a family history of an infection, disease or condition.

Further, in the sense of the present invention the expression "daily dose" means the maximum dose administered to a subject in need thereof, preferably a human subject, per day. The daily dose can be administered once daily or split into several subsets distributed over the day.

Further, in the sense of the present invention the expression "loading dose" means the administration of an initial single high(er) dose at the start of the treatment (e.g. on day 1 when the treatment is started, which is then in the course of the treatment followed by administration of lower doses of the drug (maintenance dose). Such high(er) loading dose can exceptionally exceed the herein defined daily dose amounts but preferably does not exceed the herein defined daily dose amounts.

Further, in the sense of the present invention the expression "once-only dose" means a single dose with a dose strength of the daily dose amounts defined herein, which is administered as a single-shot or a once only administration on a single day of treatment. However, a once-only treatment does not exclude follow-up treatments or follow-up once-only treatments according to the present invention following a certain time after the once-only administration, e.g. after several weeks or months.

Dose amounts defined herein generally refer to a dose calculated based on the weight of Formula (I) as the free base.

The pharmaceutical composition of the present invention preferably comprises the compound of the Formula (I), or its isotopic variants, pharmaceutically acceptable salts, co-crystals, hydrates or solvates, in a dose strength of 10 to 400 mg per single dose, more preferably in a dose strength of 10, 20, 25, 50, 100, 200 or 400 mg per single dose, even more preferably in a dose strength of 50, 100, 200 or 400 mg per single dose, and most preferred in a dose strength of 200 or 400 mg per single dose; in each case calculated based on the weight of Formula (I) as the free base.

Further, embodiments of the pharmaceutical composition of the present invention comprise the compound of the Formula (I), or its isotopic variants, pharmaceutically acceptable salts, co-crystals, hydrates or solvates, in a concentration of 0.5 to 10 mg/ml, preferably in a concentration of 0.8 or 2 mg/ml.

According to the invention the compound of the Formula (I) is preferably administered in a daily dose amount of 10 to 600 mg per daily dose, preferably 10 to 400 mg per daily dose, such as preferably in a daily dose amount of 10, 20, 25, 50, 100, 200 or 400 mg, more preferably in a daily dose amount of 50, 100, 200 or 400 mg, even more preferably in a daily dose amount of 200 or 400 mg.

Such daily dose amounts are preferably administered as single daily doses.

In a further aspect the compound of the Formula (I) is preferably administered in a dose amount of 0.05 to 10.0 mg/kg body weight, preferably 0.07 to 7.5 mg/kg body weight, more preferably 0.1 to 6.0 mg/kg body weight, such as in a dose amount of 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9 or 6.0 mg/kg body weight.

The compound of the Formula (I) can generally be formulated in parenteral administration forms as described herein comprising the total daily dose of 10 to 400 mg to be administered in one (single) dose unit (ampule, vial, infusion bag mix-o-vial, cartridge, prefilled syringe, implant (e.g. vaginal ring) etc.) to be administered as a single dose unit once daily. However, it is also possible to administer the intended daily dose via multiple parenteral dosage forms (subsets) each comprising a partial amount of the desired daily dose which in total sum up to the desired daily dose, for example by splitting a total daily dose of 200 mg into two 100 mg dose units or into four 50 mg dose units or into one 100 mg and two 50 mg dose units etc.. Such split daily dose administration regime is naturally used when administering the total daily dose on different timepoints over a day and provides high flexibility in dose finding. However, it is certainly also possible to administer a once daily treatment via two or more split sub-dose units at the same timepoint, e.g. administering a total once daily dose of 200 mg via two 100 mg dose units or via one 100 mg and two 50 mg dose units etc. at one timepoint, which offers the possibility of varying the daily total once daily dose to be administered over the treatment period easily.

In a preferred aspect of the invention the compound according to Formula (I) is administered as a single daily dose, i.e. in a once daily treatment regime.

A once daily treatment regime is particularly preferred for treatment of acute herpes simplex infections such as primary infections and during a herpes simplex outbreak or severe disease conditions such as herpes encephalitis or disseminated disease but is also particularly suitable for suppression therapy of chronic conditions.

The inventors of the invention surprisingly found that the compound of the Formula (I) surprisingly offers the possibility to be administered parenterally, i.e. intravenously, in significantly lower doses than conventional herpes simplex drugs like Acyclovir due to their higher efficacy and the potential to maintain a stable and constant high plasma level over a much longer time period.

For example, the conventional drug Acyclovir offers only low efficacy with sufficiently high plasma levels lasting for only approximately 3 hours. Therefore, Acyclovir needs to be administered in much higher total daily dose amounts of up to 1 to 4 g (BMJ Open 2020;10:e032112 and NCT03282916), which are administered orally and which must be split into multiple subsets distributed over the day to achieve lasting constantly high plasma levels, e.g. by administering 100 mg to 1 g single unit doses of Acyclovir up to 5 times per day to achieve sufficiently high plasma levels for effective treatment. But such high doses are intolerable for some patients and further such oral treatment regime requires high patient compliance and puts a high pill burden on the patient when having to swallow the medicament up to 5 times per day (see package inserts of Acyclovir and Herpes - Sexually Transmitted Infections Treatment Guidelines (https://www.cdc.gov/std/treatment-guidelines/herpes.htm).

Further, as has been described e.g. in WO2024/047508 the drug Pritelivir needs to be administered orally in daily doses of 75 mg to achieve the greatest antiviral effect but then still viral shedding remains so that further investigations in a phase II study increase the daily dose of Pritelivir to 100 mg (with a 400 mg loading dose). Pritelivir shows dose limiting toxicity in humans as doses of 400 mg led to a premature termination of the trial (Clin. Pharmacol. Drug Develop. 2023;12: 749). Surprisingly and outlined in the present application, compared to Pritelivir higher exposure and a longer half-life (t_{1/2}) were observed for **IM-250** even at lower doses (t_{1/2} from 52 to 83 h for Pritelivir versus 85 to 128 h for **IM-250** and maximum concentration (Cₘₐₓ) of 1.6 µg/mL at a of dose of 100 mg versus 2.3 µg/mL for **IM-250**).

Providing the novel parenteral (preferably intravenous) treatment regime with the parenteral pharmaceutical compositions described herein comprising the compound of the Formula (**I**) according to the present invention, in contrast, offers the possibility to administer much lower doses than required for Acyclovir of 10 to 400 mg per day due to its surprising potential to maintain a sufficiently high plasma level over several days. Further, less or extended dosing frequences are possible due to the significantly extended period of stably maintained high drug levels. For example, it was surprisingly found that an administered dose of 50 mg **IM-250** maintains the plasma level for 5 days and a 100 mg administration dose can maintain a high plasma level for 8 days, both without exhibiting dose limiting toxicity or intolerability. It was further found that, compared to Pritelivir, an improvement by the factor two can be achieved with the compounds of the present invention regarding maintaining sufficiently high and constant drug levels in the blood (plasma).

Therewith, the new administration regime according to the present invention allows not only the reduction of the total dose under toxicity and tolerability aspects but also simplifies the administration regime due to the possibility of administration once daily and / or of the possibility to extend the administration frequency for several days, which may improve patient's quality of life and patient compliance and simplifies the medical practice and routine in the hospital.

The parenteral administration route offers advantages when administered in severe cases, as high loading doses can quickly be administered and the administration can more easily be controlled by the medical practitioner, independently from patient compliance. Further, parenteral administration is more suitable for patients suffering from problems with swallowing. Further, parenteral administration benefits from quick and more direct bioavailability compared to oral administration and offers more precise application.

Based on the surprising findings the inventors made for novel dosing regimen options with the compounds of the present invention a further specific aspect of the invention relates to the use of the compound according to Formula (**I**) for the treatment as defined herein, wherein the compound of Formula (**I**) is parenterally administered as a once-only treatment, wherein the composition of the invention is administered once only per treatment of an episode. Such once only treatment can be considered as a "one shot" treatment. Preferably such once-only treatment is administered in a dose strength as defined herein for the daily dose. Therein, treatment of "an episode" means treatment of recurrent herpes simplex disease with a single dose upon onset of signs or symptoms of a recurrent infection.

A once-only treatment is particularly preferred for treatment of recurrent herpes simplex infections and can also be administered as prophylactic treatment after primary contact with herpes viruses. Further, repeated once-only treatments, e.g. monthly, can be selected for treating specific patient groups, like patients suffering from Alzheimer's disease.

Depending on the severity and the course of the disease and the patient's needs or abilities, it is also possible to administer the compositions of the invention for the use as defined herein by intermittent parenteral administration of same or different daily doses of the compound of Formula (**I**) in repeating equal or varying intervals of between 4 to 21 days, preferably between 4 to 14 days. Such intermittent therapy approaches are particularly applicable for severe / highly recurrent infections, long-time treatment and for suppression therapy to reduce viral shedding. Therein, the therapy can comprise splitting the daily dose into multiple administrations of dose subsets per day.

In one aspect, intermittent parenteral administration of the compound according to Formula (**I**) for the use according to the invention comprises the intermittent parenteral administration of same daily doses of the compound of Formula (I) in repeating equal intervals of between 4 to 14 days.

In a further aspect, intermittent parenteral administration of the compound according to Formula (**I**) for the use according to the invention comprises administration of the composition over a total treatment period of between 14 to 21 days with administration of the compound with a high single dose as an initial loading dose on the first administration day, followed by administration of the composition with a lower daily dose strength on the following days (maintenance dose).

In such specific intermittent parenteral administration regime, an initial single loading dose of 100 - 600 mg can be selected, e.g. a single loading dose of 100, 200, 400 or 600 mg, preferably of 200 to 600 mg, more preferably 200 or 400 mg, even more preferably 400 mg. The subsequent maintenance dose can be selected between 10 to 200 mg per daily dose, preferably 25 mg to 100 mg, more preferably 50 mg per daily dose.

Generally, in case of treating children or weakened patients, the herein defined daily doses can be reduced and adapted by calculating a corresponding dose based on the actual body weight of the patient to be treated.

Particularly for suppression therapy indications for the treatment or elimination of latent forms of herpes viruses in neuronal tissue and nerves and/or for the prevention and treatment of recurrence and reactivation of herpes infections or severe implications associated therewith, it is preferred to administer the compound according to Formula (**I**) by intermittent parenteral administration with dose regimens as defined herein. Such intermittent parenteral administration regimes can further be used for treating and/or curing chronic herpes infections.

A one shot or once only dosing is particularly useful for treating recurrent herpes simplex infections.

The herein defined dose amounts may be administered for the above described one shot/once only dosing as well as for chronic and intermittent administration. Depending on the severity of the disease and symptoms, the course of the disease and the physical constitution of the treated subjects suitable dose amounts can be selected.

Examples of preferred dosing amounts comprise:
- a dose amount of 50 to 400 mg, preferably 50, 100 or 200 mg, more preferably of 200 mg as a once only single daily dose for treating recurrent herpes episodes;
- a dose amount of 50 to 200 mg, preferably 50, 100 or 200 mg, as an intermittent administration for 1 to 5 days, preferably for administration every 5 to 10 days;
- a dose amount of 50 to 200 mg, preferably 50, 100 or 200 mg per daily dose, for weekly administration, preferably for a treatment period of at least 4 weeks;
- a dose amount of 10 to 200 mg, preferably 10, 20, 25, 50, 100 or 200 mg for daily administration, in particular for suppression therapy.

The novel dose regimen found by the inventors of the present invention turned out to be particularly suitable for treatment options aiming at providing a high and constant drug level in blood or plasma. Therefore, a further preferred aspect of the invention relates to the use of the pharmaceutical composition of the invention in a method of treating herpes infections, particularly herpes simplex infections, by parenteral administration of the compound according to the Formula (**I**) until a target minimum concentration in plasma or blood, preferably plasma, of at least 500 nM, preferably at least 1 µM and more preferably at least 2 µM is achieved.

Particularly for long-term treatment, e.g. of chronic conditions, a further preferred aspect of the invention relates to the use of the pharmaceutical composition of the invention in a method of treating herpes infections, particularly herpes simplex infections, by parenteral administration of the compound according to the Formula (**I**) for maintaining a target minimum concentration in plasma or blood, preferably plasma, on a steady (constant) level of at least 500 nM for at least 6 hours, preferably for maintaining a target minimum plasma concentration on a constant level of at least 1 µM for at least 24 hours, preferably for at least 2 days, more preferably for at least 3 days, more preferably for at least 4 days, even preferably for at least 5 days, much more preferably for at least 8 days, much more preferably for at least 10 days, much more preferably for at least 14 days.

For such long-term or long-lasting treatment it is particularly preferred to maintain the level of the compound according to Formula (**I**) at the target minimum plasma concentration for at least 24 hours up to 3 days, preferably for at least 24 hours up to 4 days, preferably for at least 24 hours up to 5 days, more preferably for at least 24 hours up to 8 days, more preferably for at least 24 hours up to 10 days, more preferably for at least 24 hours up to 14 days.

It is then preferred to parenterally administer the compound according to Formula (**I**) as a single dose in a once-only treatment per episode in an amount being selected to be suitable to achieve the targeted minimum plasma concentration.

As far as referred to herein, the compound plasma level or plasma concentration is determined by techniques like LC-MS/MS (liquid chromatography-tandem mass spectrometry) or HPLC (high-performance liquid chromatography).

Particularly, it is then desirable to select a dose amount suitable to achieve an essentially steady stable plasma or blood, preferably plasma, concentration level of the compound of Formula (**I**) around about 500 nM, preferably about 1 µM and more preferably about 2 µM.

Therein "around about" indicates the indicated value being as accurate as the method used to measure it and as the technical tolerance of the measurement method allows.

A further aspect of the invention relates to the use of the pharmaceutical composition of the invention in a method of treating herpes infections, particularly herpes simplex infections, by parenteral administration of the compound according to the Formula (**I**) in an amount allowing to achieve an apparent clearance rate (CL/F) of equal to or less than about 2.5 L/h, preferably equal to or less than 1 L/h more preferably less than 0.75 L/h. Therein, the clearance rate is determined by prediction of CL/F with the Phoenix software (WinNonlin) based on the plasma concentrations of a patient over time.

In a particularly preferred aspect of the invention the novel dose regimen found by the inventors of the present invention turned out to be particularly suitable for treatment options aiming at providing a high and constant drug level in neuronal tissue. Therefore, a further preferred aspect of the invention relates to the use of the pharmaceutical composition of the invention in a method of treating herpes infections, particularly herpes simplex infections, by parenteral (preferably intravenous) administration of the compound according to the Formula (**I**) until a target minimum concentration of the compound of the Formula (I) in the spinal cord of ≥ 1.0 µM, preferably ≥ 5.0 µM, more preferably ≥ 7.0 µM, more preferably ≥ 9.0 µM, even more preferably ≥ 10 µM.

Alternatively or in addition, the parenteral administration of the compound according to the Formula (I) aims at a target minimum concentration in the brain of ≥ 500 nM, preferably ≥ 1.0 µM, more preferably ≥ 5.0 µM, more preferably ≥ 7.0 µM, even more preferably ≥ 9.0 µM.

Alternatively or in addition, the parenteral administration of the compound according to the Formula (I) aims at a target minimum concentration in the trigeminal ganglia of ≥ 1.0 µM, preferably ≥ 5.0 µM, more preferably ≥ 7.0 µM, more preferably ≥ 9.0 µM, even more preferably ≥ 10 µM.

Alternatively or in addition, the parenteral administration of the compound according to the Formula (**I**) aims at a target minimum concentration in the sacral ganglia of ≥ 2.0 µM, preferably ≥ 5.0 µM, more preferably ≥ 7.0 µM, even more preferably ≥ 9.0 µM.

Alternatively or in addition, the parenteral administration of the compound according to the Formula (**I**) aims at a target minimum concentration in the cerebrospinal fluid (CSF) of ≥ 0.05 µM, preferably ≥ 0.07 µM, more preferably ≥ 0.09 µM.

The composition comprising the compound according to Formula (**I**) for the use as defined herein can be administered parenterally to the subject to be treated at between 0 to 4 days post infection or recurrence, preferably recurrence.

To achieve the aforesaid treatment regimens and desired target concentrations, the composition of the invention is preferably administered intravenously or by infusion.

### Parenteral Administration

The pharmaceutical compositions of the invention have particularly been developed to provide novel parenteral dosage forms of the compound of the Formula (**I**) and its isotopic variants, pharmaceutically acceptable salts, co-crystals, hydrates or solvates. Generally, a parenteral pharmaceutical dosage form can be provided in the form of an ampule, vial, infusion bag, mix-o-vial, cartridge, prefilled syringe or implant (e.g. vaginal ring). However, it is particularly preferred to provide parenteral dosage forms in the form of an ampule, vial, infusion bag, mix-o-vial or any other form allowing to easily administer the composition intravenously or via infusion with standard medical equipment.

The pharmaceutical composition can be a solid composition, like preferably a lyophilizate, e.g. filled in a suitable container. Generally "container" means an ampoule or vial with rubber stopper and cap, single or double chamber syringe, infusion bag or bottle made from polymeric materials or glass, suitable for housing compositions for parenteral administration. It also includes any vessel for holding liquids. In a preferred embodiment of the invention, the container is a vial, e.g. a glass vial with rubber stopper and cap.

A solid pharmaceutical composition in the form of a lyophilizate is preferred, such as particularly a lyophilizate provided in a vial for dissolution in a pharmaceutically acceptable aqueous solution, preferably a physiological solution.

If the pharmaceutical composition of the invention is provided in solid form, e.g. as a lyophilizate, a solvent is used for the reconstitution of the solid pharmaceutical composition to make it suitable for parenteral administration. A preferred solvent for reconstituting solid pharmaceutical compositions like lyophilizates is water or a physiological aqueous solution, which must be suitable for injection or infusion.

Accordingly, it is also possible to provide the pharmaceutical composition of the invention directly in liquid form for injection or infusion. The pharmaceutical composition then further comprises a pharmaceutically acceptable aqueous solution, preferably a physiological solution, more preferably an intravenous injection solution or an infusion solution. The pharmaceutical composition comprising the compound of the Formula (I) and the cyclodextrin can either be provided dissolved in such an aqueous solution to provide the injectable liquid composition directly or in solid form, e.g. as a lyophilizate together with the aqueous solution for its solubilization prior to administration e.g. in the form of a kit-of-parts combination or set. A kit-of-parts combination or set preferably comprises the pharmaceutical composition in lyophilized form, an injection vial, optionally a lyo stopper, a cap (e.g. a flip off cap), and optionally also the pharmaceutically acceptable aqueous solution, preferably physiological solution, for dissolution of the lyophilizate in the vial prior to administration, and may optionally further comprise the required infusion equipment, like a syringe, a needle, a butterfly, a winged infusion set etc. Further, a kit-of-parts may comprise the instruction manual or a leaflet and further consumables usually required in the medical practice during intravenous applications or for administering an infusion.

Therewith, the pharmaceutical composition according to the invention can be provided in the form of a ready-to-use formulation for convenient administration.

Mixing or reconstituting a solid composition prior to administration to patient is usually done not earlier than up to three days before, in particular up to 24 hours before, and for example up to 6 hours before administration to the patient.

In a particularly preferred aspect the pharmaceutical composition according to the invention comprises the compound of the Formula (I) in the form of the free base IM-250 or as its HCl salt 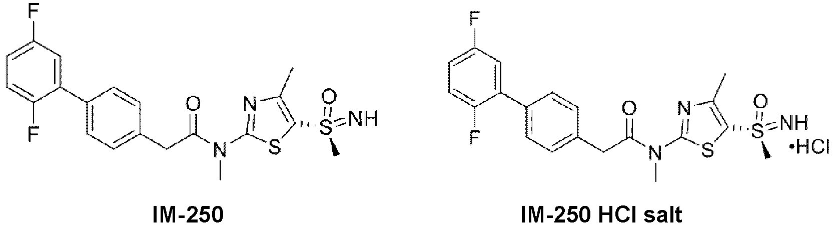
a cyclodextrin selected from HP-β-cyclodextrin and SBE-β-cyclodextrin,
a buffer, preferably Na₂HPO₄ or a hydrate thereof, more preferably Na₂HPO₄•12 H₂O, and
a pharmaceutically acceptable aqueous solution, preferably a physiological solution.

### Method of Treatment and Medical Use

The parenteral pharmaceutical composition according to the invention is intended and particularly suitable for the use as defined anywhere herein, such as particularly the use in the prophylaxis and treatment of diseases or disorders associated with viral infections caused by herpes viruses.

In the sense of the present invention prophylaxis and treatment of a disease or disorder associated with viral infections caused by herpes viruses particularly focusses on prophylaxis and treatment of diseases or disorders associated with or caused by herpes simplex viruses.

Diseases or disorders associated with or caused by herpes viruses, particularly by herpes simplex viruses, are defined in further detail below.

A medical use as defined herein can also be considered as a method of treatment characterized by the specific treatment conditions defined herein.

"Treating" and "treatment" of a disease include the following:
(1) preventing or reducing the risk of developing the disease, i.e. causing the clinical symptoms of the disease not to develop in a subject that may be exposed to or predisposed to the disease but does not yet experience or display symptoms of the disease,
(2) inhibiting the disease, i.e. arresting or reducing the development of the disease or its clinical symptoms,
(3) relieving (healing) the disease, i.e. causing regression of the disease or its clinical symptoms, and
(4) ameliorating or alleviating the symptoms or impairments caused by the disease.

"Prevention" or "preventing" or "prophylaxis" means any treatment of an infection, disease or condition that causes the clinical symptoms of the disease or condition not to develop.

The terms "subject" or "patient" refer to an animal, such as a mammal (including a human), that has been or will be the object of treatment, observation or experiment. The methods described herein may be useful in human therapy and/or veterinary applications. Preferably, the subject (or the patient) is a human. "Human (or patient) in need thereof" refers to a human who may have or is suspected to have an infection or disease or conditions that would benefit from certain treatment; for example, being treated with the compounds disclosed herein according to the present application.

Besides the general suitability for treating herpes simplex infections, due to the significant benefits the pharmaceutical compositions of the inventions offer due to the superior efficiency of the compounds of the Formula(I) to enter neuronal tissue and achieve the above defined target concentrations in spinal cord, brain, trigeminal ganglia and sacral ganglia, the pharmaceutical composition of the invention is particularly suitable for treating and reducing or eliminating latent (dormant) forms of herpes viruses in neuronal tissue, nerves and meninges, preferably for avoiding or preventing recurrence and reactivation of herpes infections or even severe implications associated therewith, such as Mollaret's meningitis or herpes simplex encephalitis (HSE).

In addition, the pharmaceutical compositions of the inventions can be used in the prophylaxis or treatment of neurodegenerative diseases caused by viruses, such as in particular Alzheimer's disease caused by viruses, in particular caused by Herpes simplex viruses.

Therefore, the pharmaceutical compositions of the inventions can be administered for the prophylaxis or treatment of herpes infections, in particular Herpes simplex infections, in patients displaying Herpes labialis, Herpes genitalis and Herpes-related keratitis, Herpetic esophagitis, Herpetic pneumonia, Alzheimer's disease, encephalitis, Mollaret's meningitis, pneumonia, hepatitis; in patients with a suppressed immune system, such as AIDS patients, cancer patients, patients having a genetic immunodeficiency, transplant patients; in new-born children and infants; in Herpes-positive patients, in particular Herpes-simplex-positive patients, for suppressing recurrence or viral shedding (suppression therapy); or in patients, in particular in Herpes-positive patients, in particular Herpes-simplex-positive patients, who are resistant to nucleosidic antiviral therapy such as Acyclovir, Penciclovir, Famciclovir, Ganciclovir, Valacyclovir and/or compounds such as the phosphonates Foscarnet or Cidofovir.

In principle, the use of the pharmaceutical composition of the invention as described herein can be considered for the use in the prophylaxis and treatment of the respective disorders and diseases in humans as well as in animals, although the treatment of humans is preferred.

Therefore, the invention relates particularly to a method of preventing or treating a disease or disorder associated with viral infections, such as a disease or disorder, which is associated with viral infections caused by herpes viruses, such as in particular by Herpes simplex viruses as well as a method of treating and eliminating latent (dormant) forms of herpes viruses in neuronal tissue and nerves, preferably for avoiding or preventing recurrence and reactivation of herpes infections or even severe implications associated therewith, such as herpes simplex encephalitis (HSE) or Mollaret's meningitis, or a method of preventing or treating neurodegenerative diseases caused potentially by viruses, such as in particular Alzheimer's disease, by parenterally administering to a subject in need thereof the pharmaceutical compositions of the inventions comprising an effective amount of the compound of the Formula (**I**), optionally with a treatment regime as described herein.

More particularly, the invention relates to a method of preventing or treating diseases caused by or associated with Herpes simplex infections, which are selected from encephalitis, neonatal herpes esophagitis and Alzheimer's disease, by parenterally administering to a subject in need thereof the pharmaceutical compositions of the inventions comprising an effective amount of the compound of the Formula (**I**), optionally with a treatment regime as described herein.

### Combination Therapy

The compounds of the present invention may also be used in a combination therapy for the prophylaxis and treatment as defined herein. A combination therapy comprises co-administration of the compounds of the Formula (I) in the form of the pharmaceutical composition of the present invention in combination with one or more further active ingredients ("co-drug(s)"). Preferably, such combination therapy comprises co-administration with one or more active ingredients exhibiting advantageous effects in the treatment of the disorders or diseases as described herein and associated conditions, including e.g. antiviral compounds, vaccines and immune modulators, e.g. glucocorticoids. More particularly a combination therapy comprises co-administration with one or more further active substance being effective in treating a disease or disorder associated with viral infections (antiviral active compounds), preferably a disease or disorder being associated with viral infections caused by herpes viruses, such as in particular by Herpes simplex viruses.

The at least one further active substance being effective in treating a disease or disorder associated with viral infections or more preferably antiviral active compounds can be selected from the group consisting of nucleosidic drugs such as Acyclovir, Valacyclovir, Penciclovir, Ganciclovir, Famciclovir and Trifluridine, as well as from the compounds Foscarnet and Cidofovir. It is also possible to select such potential co-drugs from the group of helicase-primase inhibitors known from the prior art, e.g. Pritelivir or the above-described "Compound 1" (ABI-5366) disclosed in WO2024/224304 or the helicase-primase inhibitor ABI-1179 currently under development in clinical trials.

A combination therapy can comprise co-administration of the compounds of the Formula (I) and the above defined co-drug(s) in a fixed dose or free dose combination for sequential use.

Combination therapy in a fixed dose combination therapy comprises co-administration of the compounds of the Formula (I) with the at least one additional pharmaceutically active compound (co-drug) in a fixed-dose formulation. Such fixed-dose formulations relate to a pharmaceutical composition of the present invention which additionally comprises the co-drug, formulated together with the compounds of the Formula (I) and the cyclodextrin in the same galenic formulation.

Combination therapy in a free dose combination therapy comprises co-administration of the compounds of the Formula (I) with the at least one additional pharmaceutically active compound (co-drug) in free doses of the respective compounds, either by simultaneous administration of the individual compounds or by sequential administration of the individual compounds distributed over a time period. Therein, simultaneous administration covers providing the pharmaceutical composition of the invention comprising the compounds of the Formula (I) and providing a second pharmaceutical composition comprising the co-drug (e.g. Acyclovir) separately and mixing both prior to administration to the patient, e.g. by adding both to an infusion bag.

A combination therapy in a free dose combination therapy can further comprise the parenteral administration of the pharmaceutical composition of the present invention in combination with an oral administration of antiviral compounds as described herein. Such specific form of free dose combination therapy may further cover parenteral administration of the pharmaceutical composition of the present invention comprising the compounds of the Formula (I) in combination with an oral administration of dosage forms also comprising the compounds of the Formula (I) of the present invention.

Accordingly, a further aspect of the present invention relates to a pharmaceutical composition comprising the compound of the Formula (I) as described herein and at least one further active substance being effective in treating a disease or disorder associated with viral infections (antiviral active compounds) as defined herein. Preferred co-drugs are selected from active substances being effective in treating herpes (simplex) infections, among which Acyclovir is the most preferred co-drug in a combination therapy.

Further preferred combination therapy options comprise combinations of administering different dosage forms of compounds of the Formula (I), e.g. combining parenteral with oral administration regimes.

### Process for Preparing the Pharmaceutical Composition

A further aspect of the invention relates to the process for the preparation of the pharmaceutical composition according to the invention as described herein. Such a process comprises the following steps:
(a) preparing a solution of one or more cyclodextrins in water to obtain an aqueous cyclodextrin-solution and optionally adjusting the pH to an acidic pH of < 5.0, preferably pH ≤ 4.0, more preferably pH ≤ 3.0, even more preferably pH ≤ 2.0,
(b) adding and solubilizing the compound of the Formula (I), or its isotopic variant, pharmaceutically acceptable salt, co-crystal, hydrate or solvate, in the cyclodextrin-solution of step (a) so that the resulting solution has an acidic pH of < 5.0, preferably pH ≤ 4.0, more preferably pH ≤ 3.0, even more preferably pH ≤ 2.0,
(c) subsequently adding a pH adjusting agent, e.g. a buffer, to the solution of step (b) and increasing the pH of the solution to a pH of 6.0 to 7.0, preferably 6.1 to 6.9, more preferably 6.2 to 6.8, more preferably 6.3 to 6.7, most preferred 6.5,
(d) freeze-drying of the solution of step (c) to obtain a lyophilizate.

For adding and solubilizing the compound of the Formula (I) in the cyclodextrin-solution of step (a) an acidic pH is required, i.e. a pH of < 5.0, preferably pH ≤ 4.0, more preferably pH ≤ 3.0, even more preferably pH ≤ 2.0.

The control of the acidic pH can be achieved by adding suitable pH adjusting agents, like acids, comprising for example hydrochloric acid (HCl), citric acid, acetic acid, phosphoric acid, preferably hydrochloric acid (HCl).

If the pH of the cyclodextrin-solution of step (a) is > 5.0 when adding the compound of the Formula (I) according to step (b), then the compound of the Formula (I) precipitates and forms a solid slurry which remains insoluble and no clear solution can be provided.

If in step (b) the compound of the Formula (I) is added in the form of the HCl salt, usually no or only slight pH adjustment of the cyclodextrin-solution is necessary. In particular, when adding the compound of the Formula (I) in the form of the HCl salt to a cyclodextrin-solution, e.g. of the preferred cyclodextrins HP-β-cyclodextrin and/or SBE-β-cyclodextrin, no pH adjustment is necessary as the HCl salt addition leads to a sufficiently acidic pH solution by itself.

Accordingly, using the HCl salt of the compound of the Formula (I) is preferred.

If in step (b) the compound of the Formula (I) is added in the form of the free base, pH adjustment of the cyclodextrin-solution to acidic pH is necessary prior to adding the compound of the Formula (I).

After mixing the compound of the Formula (I) into the cyclodextrin-solution and complete dissolution therein to obtain a clear solution, a pH adjusting agent, e.g. a buffer, is added to this clear solution to increase the pH to physiological pH values, e.g. pH values in the above defined ranges.

Therein, any suitable and pharmaceutically acceptable pH adjusting agent can be used, like NaHCO₃, acetic acid, hydrochlorid acid (HCl) or a suitable buffer, e.g. a buffer selected from acetic acid/sodium acetate, Na₂HPO₄ or a hydrate thereof, like Na₂HPO₄•12 H₂O.

Preferably a buffer is added in step (c), such as Na₂HPO₄ or a hydrate thereof, particularly Na₂HPO₄•12 H₂O.
The so obtained solution is then subjected to freeze-drying to obtain a lyophilizate. Such lyophilizate can provide the pharmaceutical composition of the invention in solid form.

If a liquid pharmaceutical composition, e.g. an injectable solution, shall be prepared, the process of the invention can further comprise the following additional step (e) of adding a pharmaceutically acceptable aqueous solution, preferably a physiological solution, more preferably an intravenous injection solution or an infusion solution, to the lyophilizate obtained in step (d) to provide a liquid injectable parenteral pharmaceutical composition according to the invention.

Preferably, in step (e) the pharmaceutically acceptable aqueous solution, preferably the physiological solution, more preferably the intravenous injection solution or infusion solution, is added to the lyophilizate obtained in step (d) in an amount suitable to dilute the lyophilizate completely and in an amount to obtain the target concentration of the compound of the Formula (I) in the solution, e.g. in the injectable parenteral pharmaceutical composition.

If the pharmaceutical composition contains additional excipients, auxiliaries, carrier or an optional co-drug(s) (like preferably Acyclovir) said additional components can be added in either of the process steps (a) to (c) and (e). It is also possible to add one or more of said additional components in the same or in different process steps described above.

As mentioned above, preferred additional excipients are selected from buffer, preferably Na₂HPO₄ and a hydrate thereof, more preferably Na₂HPO₄•12 H₂O, and from tonicity adjusting agents, preferably selected from glucose and sodium chloride, more preferably sodium chloride. For a combination therapy formulation as described above the additional active substances are preferably selected among those being effective in treating herpes infections, more preferably selected from antiviral drug compounds, even more preferably Acyclovir.

It is possible, although not critical, to add the compound of the Formula (I), or its isotopic variants, pharmaceutically acceptable salts, co-crystals, hydrates or solvates, in step (b) in micronized form.

Therein, micronized forms of the compound of the Formula (I) are preferably characterized by a d₉₀ value of less than or equal to about 20.0 µm. More preferably, micronized forms of compounds of the Formula (**I**) are characterized by a d₉₀ value of less than or equal to about 10.0 µm.

In the context of the present invention, micronized forms of the compound of the Formula (**I**) refer to micronized forms as already described in WO2025/017032.

If a micronized form is used, it is preferably a micronized form having a d₉₀ value of less than or equal to about 20.0 µm, more preferably micronized with a d₉₀ value of less than or equal to about 10.0 µm.

Particularly, as defined therein, the term "d₉₀ value" is a percentile value, which refers to 90% (by volume) of the particles have a size that is less than or equal to the value. For example, d₉₀ value of 20.0 µm means 90% (by volume) of the particles is less than or equal to 20.0 µm in size; a d₉₀ value of 10.0 µm means 90% (by volume) of the particles is less than or equal to 10.0 µm in size. The "d₉₀ value" can be derived from a particle-size distribution (PSD) assessment by laser diffraction (granulometry analysis). Similarly, the terms "d₁₀", "d₂₅", "d₅₀" and/or "d₇₅" define the respective percentage (by volume) of the particles having a size that is less than or equal to the value. The term "d₉₀ particle size distribution" means that 90% (per volume) of the particles have a particle size lower than the d₉₀ value expressed in µm. Similarly, the terms "d₁₀ particle size distribution", "d₂₅ particle size distribution", "d₅₀ particle size distribution" and/or "d₇₅ particle size distribution" mean that 10%, 25%, 50% and/or 75% (per volume) of the particles have a particle size above or below the respectively defined d₁₀, d₂₅, d₅₀ and/or d₇₅ value expressed in µm. These d-values relate in particular to the cumulative particle volume in the particle distribution curve. The determination of the particle size distribution is carried out as described in Example 7 of WO2025/017032 and micronization of the compounds of the Formula (**I**) can likewise be carried out as described therein.

The lyophilization conditions in step (d) can be controlled in accordance with a skilled person's practical knowledge. In a particularly preferred aspect, the invention relates to a process for the preparation of a pharmaceutical composition in the form of a lyophilizate, wherein the step (d) comprises the following lyophilization condition steps (which are, however, not mandatory for the process in general):
(i) transferring the solution of step (c) into a lyophilization chamber in suitable open containers, e.g. vials, at about room temperature and normal atmospheric pressure,
(ii) cooling the solution of step (c) to a temperature between -20°C and -80°C, preferably between -40°C and -55°C, more preferably to about -45°C,
(iii) subjecting the cooled solution of step (ii) to a primary drying phase to obtain a primarily dried pharmaceutical composition, wherein the chamber is subjected to a vacuum of 0.05 mbar to 2 mbar, preferably 0.1 mbar to 0.6 mbar, more preferably to about 0.2 mbar at the temperature as set in step (ii), preferably carrying out said primary drying under a controlled ramp rate, wherein the temperature is increased to 10°C over a period of 10 to 50 h, preferably over a period of about 30 h,
(iv) subjecting the primarily dried pharmaceutical composition to a secondary drying phase, wherein the temperature is increased to between 20°C and 40°C, preferably to 35°C, at the vacuum as set in step (iii) to remove any residual moisture, and
(v) subjecting the so obtained lyophilizate to a final processing by removing the lyophilized pharmaceutical composition from the lyophilization chamber and capping the container, e.g. the vial.

The pharmaceutical composition prepared according to the process described anywhere herein can then further be packed and stored until use.

### DESCRIPTION OF FIGURES

- **Figure 1:**: Plasma concentration of **IM-250** according to Example 3 - Part A in Sprague-Dawley rats.
- **Figure 2:**: Drug concentrations of HPIs in the brain of mice and the corresponding brain/plasma ratios (brain/plasma ratio = bars; drug concentration brain = dots)
- **Figure 3:**: Brain/plasma ratios of IM-250 in diverse species at defined time points after administering diverse doses orally or intravenously either as single (SD) or multiple doses (MD) over several days
- **Figure 4:**: Exposure of IM-250 in diverse tissues of beagle dogs after multiple dosing of 100 mg/kg once daily for 8 consecutive days 24 hours after last administration (IM-250 concentration = bars; tissue/plasma ratio = dots)
- **Figure 5:**: Exposure of IM-250 in nervous tissues of SD rats after a single dose of 3 mg/kg IM-250 4 hours after administration (bars from left to right correspond to legend from top to bottom)

### EXPERIMENTAL PART

Methods for the preparation of the compound **IM-250** are known and have been described for example in Example 7(-) of WO2019/068817 or in WO2023/135303.

A preferred crystalline form of the **IM-250 HCl salt** referred to herein shows a XRPD pattern with the following XRPD peaks (Table 1).

**Table 1: XRPD Peak positions (°2Θ) and intensities**

| **°2-Theta (°2Θ)** | **Relative intensity (%)** |
|---|---|
| 9.1 | 24 |
| 11.2 | 13 |
| 11.8 | 20 |
| **13.7** | **100** |
| 14.1 | 17 |
| 16.4 | 23 |
| **17.0** | **55** |
| 17.3 | 21 |
| **17.7** | **98** |
| 18.2 | 34 |
| 19.4 | 26 |
| **19.8** | **42** |
| 21.1 | 23 |
| 21.3 | 33 |
| **21.8** | **33** |
| 22.6 | 15 |
| **22.8** | **62** |
| 23.4 | 11 |
| 23.7 | 11 |
| 24.0 | 15 |
| 24.2 | 16 |
| 25.0 | 30 |
| 26.3 | 35 |
| 26.9 | 12 |
| 27.1 | 19 |
| 27.4 | 11 |
| 27.7 | 22 |
| 32.0 | 11 |

As described above, **IM-250** (molecular weight 435.5), in both its free base form and its hydrochloride salt **IM-250 HCl** (molecular weight 472.0) can be formulated as both share the same mechanism of action. In the following table the amount (in mg) of the compound is converted into µmol.

| **IM-250 [µmol]** | **IM-250 [mg]** | **IM-250 HCl salt [mg]** |
|---|---|---|
| 23.0 | 10.0 | 10.8 |
| 45.9 | 20.0 | 21.7 |
| 57.4 | 25.0 | 27.1 |
| 115 | 50.0 | 54.2 |
| 230 | 100 | 108 |
| 459 | 200 | 217 |
| 918 | 400 | 434 |

Similar calculations can be made with the deuterated form.

### Example 1: Preparation and Characterization of a Pharmaceutical Composition according to the Invention for Intravenous Administration (Infusion)

In accordance with a preferred aspect of the invention a liquid, ready-to-use intravenous formulation for the helicase-primase inhibitor **IM-250** was developed. Therein, the **IM-250 HCl** was used for the preparation of a formulation with a targeted dose strength of 50 to 200 mg at a target concentration of 1 to 2 mg/mL, using 100 R vials allowing storage at room temperature with a target shelf life (2 to 5 years). Alternatively, a 20 R vial can be used at 10 mg/mL (diluted 1 to 2 mg/mL in 0.9% NaCl or 5% glucose).

### Part A - Formulation screening:

First, a suitable method to solubilize **IM-250 HCl** at a target concentration of 2 mg/ml was developed. The salt factor of 1.084 was not taken into account.

The following solubilization approaches were tested:

| **#** | **Excipients** | **Preparation** | **Solubility** |
|---|---|---|---|
| 1 | Lipofundin MCT 20% | Dissolve **IM-250 HCl** at 2 mg/ml in Lipofundin MCT 20% | Partially |
| 2 | Liposomes | 1) Dissolve **IM-250 HCl** at 10 mg/ml in 25% Lecithin S100 in propylene glycol | Yes |
| | | 2) Dilute 1/5 in purified water | |
| 3 | Mixed micelle/3.5% sodium cholate, 3% lecithin (S100, soja) in water for injection at pH 7.0 | Dissolve **IM-250 HCl** at 2 mg/ml in mixed micelle solution | Partially |
| 4 | 5% (w/w) HP-β-cyclodextrin (CD) in purified water | Dissolve **IM-250 HCl** at 2 mg/ml in HP-β-CD solution | Yes |
| 5 | 20% propylene glycol | Dissolve **IM-250 HCl** at 2 mg/ml in 20% propylene glycol | No |
| 6 | Liposomes with polysorbate | 1) Dissolve **IM-250 HCl** at 10 mg/ml in 0.5% PS20, 25% Lecithin S100 in propylene glycol | Yes |
| | | 2) Dilute 1/5 in purified water | |
| 7 | 5% (w/w) recombinant human serum albumin (HSA) in purified water | Dissolve **IM-250 HCl** at 2 mg/ml in HSA solution | Partially |
| 8 | 300 mM proline | Dissolve **IM-250 HCl** at 2 mg/ml in proline solution | No |
| 9 | Capmul | 1) Dissolve **IM-250 HCl** at 40 mg/m in Capmul | No |
| | | 2) Dilute 1/20 in purified water | |
| 10 | aqueous solution | Dissolve **IM-250 HCl** at 2 mg/ml in purified water | No |

After compounding the samples, they were incubated for at least 2 h to allow maximal solubility. Afterwards the samples were first centrifugated for 5 min at 4000 rpm to remove insoluble particles. Promising variants were passed over a sterile filter before analyzing by reverse-phase high-performance liquid chromatography (RP-HPLC) for concentration and purity.

(2-Hydroxypropyl)-β-cyclodextrin (MS=0.4-1.5) (HP-β-CD) turned out to be the most promising excipient to solubilize **IM-250 HCl.** Afterwards the maximum solubility of two different types of cyclodextrins (HP-β-CD and SBE-β-CD) and the minimum molar ratio between cyclodextrin and **IM-250 HCl** to fully solubilize the drug at highest concentration and at 2 mg/ml were determined.

Complete solubility of **IM-250 HCl** was obtained for the liposomal formulation #2, HP-β-CD formulation #4 and the liposomal formulation with polysorbate 20 #6. Partial solubility was obtained for Lipofundin #1, the mixed micelle formulation #3 and the HSA formulation #7. All of these variants were also filtrable through a sterile filter. These samples were analyzed after preparation and after storage for 24 h at rt. The results of the RP-HPLC regarding concentration of the solutions and purity of the samples are:

| **#** | **Formulation** | **t = 0 h: concentration / relative peak area IM-250** | **t = 24 h: concentration / relative peak area IM-250** |
|---|---|---|---|
| 3 | Mixed micelle | 1.48 mg/ml / 97.8% | 1.47 mg/ml / 98.2% |
| 4 | 5% (w/w) HP-β-CD in purified water | 2.08 mg/ml / 98.9% | 2.09 mg/ml / 98.7% |
| 7 | 5% (w/w) recombinant HSA in purified water | 0.39 mg/ml / 97.9% | 0.40 mg/ml / 97.5% |

The highest solubility was achieved in the cyclodextrin variant #4 followed by the mixed micelle formulation #3 and the HSA formulation #7. Purity of the samples was comparable to the reference material direct after preparation and after storage for 24 h at rt.

From these results, cyclodextrin was identified as the most promising excipient for developing a suitable formulation. To define the borders of possible drug product (DP) presentations (concentrated solution, which is diluted before administration or "ready to infuse" presentation) the maximum solubility of cyclodextrin and **IM-250 HCl** in these solutions was determined. Additionally, the minimum molar ratio of **IM-250 HCl** to cyclodextrin at low concentrations of **IM-250 HCl** suitable for direct infusion was defined.

For both types of cyclodextrin tested (HP-β-CD and SBE-β-CD), solutions of 50% (w/w), 33.3% (w/w) and 25% (w/w) were prepared. Clear solutions were obtained at cyclodextrin concentrations of 25% (w/w) for both cyclodextrin types tested (HP-β-CD (Cavasol^{®} W7 HP from Wacker Chemie AG, Burghausen, Germany) and SBE-β-CD (Dexolve7 from Cyclolab, Budapest, Hungary).

In a second experimental setup, the minimum amount of a 25% (w/w) cyclodextrin solution is needed to dissolve 200 mg of **IM-250 HCl.** For both cyclodextrin types tested (HP-β-CD and SBE-β-CD), 7.0 g of a 25% (w/w) cyclodextrin solution were needed to dissolve 200 mg **IM-250 HCl.** This corresponds to maximum solubility of about 28 mg/g **IM-250 HCl** and a molar ratio between **IM-250 HCl** and cyclodextrin of about 1:3 (**IM-250 HCl** to HP-β-CD).

For the evaluation of the lower **IM-250 HCl** concentration, the prepared solutions were diluted with saline to 2 mg/ml **IM-250 HCl.** In contrast to SBE-β-CD, precipitation was observed after dilution for HP-β-CD. The formed complex of HP-β-CD seemed to be weaker compared to SBE-β-CD, leading to a need for an increase of the HP-β-CD concentration, which results in a clear solution at a molar ratio of 1:4 to 1:5 (**IM-250 HCl** to HP-β-CD). The solutions at both concentrations of **IM-250** were analyzed by RP-HPLC after preparation regarding concentration and purity. The results are:

| **#** | **Formulation** | **concentration / relative peak area IM-250** |
|---|---|---|
| A | High concentration **IM-250 HCl** in HP-β-CD (molar ratio 1:3) | 28.2 ± 0.1 mg/ml / 98.9 ± 0.0% |
| B | High concentration **IM-250 HCl** in SBE-β-CD (molar ratio 1:3) | 28.3 ± 0.1 mg/ml / 98.8 ± 0.0% |
| C | Low concentration **IM-250 HCl** in HP-β-CD (molar ratio 1:5) | 2.0 ± 0.0 mg/ml / 98.8 ± 0.0% |
| D | High concentration **IM-250 HCl** in SBE-β-CD (molar ratio 1:3) | 2.0 ± 0.0 mg/ml / 98.8 ± 0.3% |

All of the formulations exhibited the theoretical concentration of **IM-250.** The purity of the samples was comparable to the reference standard.

### Conclusion:

The Example surprisingly shows that most promising results for hydrophobic **IM-250 HCl** were obtained for cyclodextrin (CD) formulation variants. The highest concentration of **IM-250** was determined to be 28 mg/mL in both tested cyclodextrin types at a molar ratio of 1:3 (**IM-250 HCl** to CD). With SBE-β-CD **IM-250** forms a more stable complex, as no precipitation was observed after dilution of the concentrated solution to 2 mg/mL, which is a suitable concentration of **IM-250 HCl** for infusion. For HP-β-CD the molar ratio between **IM-250 HCl** and CD must be enhanced to 1:4 to 1:5 to prevent precipitation after dilution.

### Part B - Preclinical Batch Manufacturing:

Two 200 mg **IM-250** (in the form of **IM-250 HCl**) non-GMP batches for animal testing have been prepared:

| | **Batch 1** | **Batch 2** | **Batch 3 (placebo)** |
|---|---|---|---|
| **IM-250** (free base) | 200 mg | 200 mg | |
| **IM-250 HCl** (salt factor: | (10 mg/ml) | (10 mg/ml) | N/A |
| 1.084 incl. purity 1.094) | 218.8 mg | 218.8 mg | |
| HP-β-CD | 3000 mg | N/A | 3000 mg |
| SBE-β-CD | N/A | 2660 mg | N/A |
| water for injection | ad 20 ml | ad 20 ml | ad 20 ml |

Representative procedure (Batch 1): To HP-β-CD (Cyclolab: MS = 0.4-1.5); 64.5 g) was added distilled water (344 mL) in a sterile environment and the mixture was stirred at rt to homogeneity. Then **IM-250 HCl** (4.7 g) was added and again stirred to homogeneity and additional distilled water (86 mL) was added and the pH was adjusted to pH = 6.6 with 30% aq. NaOH. Under laminar flow condition the formulation was aseptically filtered through a 0.2 µm PVDF membrane filter (Mini Kleenpak Capsule, Fluorodyne II membrane, 0.2 µm) and filled in 20 ml vials (20R vial, type I glass), closed with serum stoppers and crimped. The other batches were prepared similarly using the appropriate reagents.

### Part C - Filtration Study:

A filtration study of the DP was performed using polyethersulfon (PES) inline-filter and polyvinylidene fluoride (PVDF) membrane filters. Samples were passed over the syringe filters and analyzed before and after filtration with regards to concentration and purity by RP-HPLC, as well as turbidity by nephelometry and pH. Additionally, the osmolality of the DP solutions was determined.

The analytical results of the preclinical test samples (from Part B) are as follows:

| | **Batch 1 (HP-β-CD)** | **Batch 2 (SBE-β-CD)** |
|---|---|---|
| Concentration of **IM-250** by RP-HPLC | n1: 9.22 mg/ml | n1: 9.46 mg/ml |
| | n2: 9.84 mg/ml | n2: 9.93 mg/ml |
| Purity of **IM-250** by RP-HPLC | n1: 97.11% | n1: 97.44% |
| | n2: 97.12% | n2: 97.44% |
| Turbidity | 2.99 NTU | 6.58 NTU |
| pH | 5.65 | 5.84 |
| Osmolality | 182 mOs/kg | 421 mOs/kg |

The concentration of the two prepared batches was within the target range of 10 mg/ml ± 10%.

The purity was slightly lower compared to the samples with cyclodextrin in Part B, because a hydrophilic shoulder occurred in the RP-HPLC chromatogram. This is may be caused by the pH adjustment of the solutions to more neutral pH values. Turbidity and pH were in the desired range. The osmolality of the SBE-β-CD sample was higher compared to the HP-β-CD as the SBE-β-CD raw material was a sodium salt.

The results of the filtration study are as follows:

| | | **Before filtration** n1/n2 | **After filtration with PES** n1/n2 | **After filtration with PVDF** n1/n2 |
|---|---|---|---|---|
| Concentration of **IM-250** by RP-HPLC [mg/ml] | Batch 1 | 9.22/9.84 | 9.31/9.77 | 9.32/9.83 |
| | Batch 2 | 9.46/9.93 | 9.46/9.81 | 9.40/9.55 |
| Purity of **IM-250** by RP-HPLC [%] | Batch 1 | 97.11/97.12 | 97.04/96.98 | 97.08/97.13 |
| | Batch 2 | 97.44/97.44 | 97.52/97.64 | 97.58/97.48 |
| Turbidity [NTU] | Batch 1 | 2.99 | 3.00 | 3.48 |
| | Batch 2 | 6.58 | 5.46 | 2.88 |
| pH | Batch 1 | 5.65 | 5.64 | 5.60 |
| | Batch 2 | 5.84 | 5.66 | 5.61 |

No differences were observed in the parameters investigated before and after filtration with PES and PVDF membrane filters for both batches of IM-250 complexed with CDs, respectively.

The turbidity of batch 2 was slightly higher before filtration and after filtration with PES, but the turbidity value is still very low (acceptance criteria according to Ph. Eur is below 30 NTU). DP sterile filtration with commonly used filter material is possible with both DP compositions.

### Part D - Infusion Media Compatibility Study:

Infusion media compatibility was tested for both **IM-250** batches from Part C. The samples were diluted to 2 mg/ml (100 ml infusion bag) and 0.8 mg/ml (250 ml infusion bag) using two types of infusion media (0.9% saline and 5% glucose infusion media, respectively). Samples were visually examined for precipitation before the samples were passed over an infusion line filter equipped with a PES membrane filter. After filtration, the samples were analyzed with regard to concentration and purity by RP-HPLC, as well as turbidity by nephelometry (using a 2100AN turbidity meter (Hach Lange, Düsseldorf, Germany) according to the European Pharmacopeia) and pH (using a calibrated SevenMulti pH meter). Additionally, the osmolality of the solutions was determined by measuring the freezing point depression of the samples using a calibrated osmometer.

All samples remained clear after dilution with infusion media. The analytical results after filtration are as follows:

| | Concentration of **IM-250** by RP-HPLC [mg/ml] | Purity of **IM-250** by RP-HPLC [%] | Turbidity [NTU] | pH | Osmolality [mOs/kg] |
|---|---|---|---|---|---|
| Batch 1 HP-β-CD 2 mg/mL in saline | 1.87 | 97.51 | 3.46 | 5.77 | 277 |
| | 1.88 | 97.49 | | | |
| Batch 1 HP-β-CD 0.8 mg/mL in saline | 0.68 | 97.45 | 3.64 | 6.01 | 286 |
| | 0.68 | 97.46 | | | |
| Batch 1 HP-β-CD 2 mg/mL in glucose | 1.87 | 97.51 | 3.41 | 5.80 | 278 |
| | 1.91 | 97.51 | | | |
| Batch 1 HP-β-CD 0.8 mg/mL in glucose | 0.70 | 97.59 | 3.32 | 5.63 | 290 |
| | 0.71 | 97.51 | | | |
| Batch 2 SBE-β-CD 2 mg/mL in saline | 1.87 | 97.52 | 3.86 | 5.83 | 309 |
| | 1.89 | 97.45 | | | |
| Batch 2 SBE-β-CD 0.8 mg/mL in saline | 0.71 | 97.41 | 3.65 | 6.08 | 301 |
| | 0.71 | 97.45 | | | |
| Batch 2 SBE-β-CD 2 mg/mL in glucose | 1.92 | 97.52 | 3.22 | 5.82 | 320 |
| | N/A | N/A | | | |
| Batch 2 SBE-β-CD 0.8 mg/mL in glucose | 0.71 | 97.53 | 3.71 | 5.68 | 307 |
| | 0.72 | 97.54 | | | |

All parameters investigated were within the desired range. The concentration was slightly lower than target, but matches the expected values, when the measured concentration of the DP is considered. The expansion of both DP batches in 0.9% saline and 5% glucose infusion media at **IM-250** concentrations of 2 mg/mL and 0.8 mg/mL is possible.

### Part E - Short-Term Stability Study:

The test was performed with the DP material at 2 to 8°C and 40°C for 4 weeks. Samples were stored upside-down. After storage for 4 weeks some visible particles were observed in the samples independent of the storage temperature. More particles were observed in batch 2 containing SBE-β-CD.

The analytical results of the stability samples are as follows:

| | | **T0** | **Storage for 4 weeks at 2-8°C** n1/n2 | **Storage for 4 weeks at 40°C** n1/n2 |
|---|---|---|---|---|
| Concentration of **IM-250** by RP-HPLC [mg/ml] | Batch 1 | 9.22/9.84 | 9.53/10.00 | 9.35/9.97 |
| | Batch 2 | 9.46/9.93 | 9.66 /10.13 | 9.64/9.55 |
| Purity of **IM-250** by RP-HPLC [%] | Batch 1 | 97.11/97.12 | 96.94/96.91 | 94.60/94.61 |
| | Batch 2 | 97.44/97.44 | 97.19/97.16 | 94.76/94.77 |
| Turbidity [NTU] | Batch 1 | 2.99 | 4.04/3.57 | 4.56/4.62 |
| | Batch 2 | 6.58 | 6.95/7.82 | 7.85/8.29 |
| pH | Batch 1 | 5.65 | 5.52/5.53 | 4.58/4.55 |
| | Batch 2 | 5.84 | 5.53/5.54 | 4.75/4.74 |

The concentration of the samples remained constant throughout the stability study, independently of the used cyclodextrin type or the storage temperature. The relative main peak area by RP-HPLC dropped approx. 0.2% at storage temperatures of 2 to 8°C, independently of the cyclodextrin type used. At 40°C storage temperature, a drop in purity of approx. 2.5% was observed. The drop in purity was independent of the cyclodextrin type used.

The turbidity of the samples remained unchanged during the stability study.

A drop in pH was observed in samples stored at 40°C, which might be attributed to the decay of the DP towards 2-[4-(2,5-difluorophenyl)phenyl]acetic acid.

### Conclusion:

**IM-250 HCl** can be formulated as an iv-formulation in HP-β-CD and SBE-β-CD (sulfobutylether-β-cyclodextrin) at a concentration of 10 mg/ml (20 ml 20R vials containing 200 mg **IM-250** = 218.8 mg **IM-250 HCl**, complexed with 3000 mg HP-β-CD (15%, 150 mg/ml) or 2660 mg SBE-β-CD (13.3%, 133 mg/ml), respectively).

The expansion of both DP batches in 0.9% saline and 5% glucose infusion media at **IM-250** concentrations of 2 mg/ml (100 ml infusion bag) and 0.8 mg/ml (250 ml infusion bag) is possible and compatible with in-line 0.2 µm PES and PVDF filters during infusion.

The pH of the dissolved API is low (pH ~ 2) and had to be adjusted with NaOH to pH 5 to 6. A sodium phosphate buffer is recommended, since the API is sensitive to hydrolysis at pH >9.0. A short-term stability study showed the formation of visible particles after storage for 4 weeks both at 2 to 8°C and 40°C. More particles were observed in batch 2 containing SBE-β-CD.

The concentration of **IM-250** remained constant for both CDs, however, the purity declined during 4 weeks storage at 40°C. RP-HPLC showed degradation at a storage temperature of 40°C in both HP-β-CD and SBE-β-CD samples (drop in rel. main peak area by ~2.5%). The turbidity of the samples remained unchanged and the pH dropped at 40°C from 5.5 to 4.5 which might be attributed to formation of the decay product 2-[4-(2,5-difluorophenyl)phenyl]acetic acid. The addition of buffer (e.g. Na₂HPO₄) is therefore recommended.

### Example 2: Thermal Characterization and Critical Pressure Scanning (CPS), Preparation and Lyophilization Cycle Development

To extend the limited stability (3 months) of **IM-250** in the liquid formulations of Example 1, a lyophilization cycle for the lyophilized formulation for **IM-250 HCl** at 10 mg/ml (**IM-250 free base**) for i.v. application (infusion) was developed.

The glass transition temperature Tg' for the drug product (DP) solution was determined by differential scanning calorimetry using a DSC-8000 (Perkin Elmer, Waltham, MA, USA). Approx. 20 mg of the corresponding lyo solution were weighed into a 50 µL aluminum sample pan. The pan was sealed with a closed aluminum DSC-cover. An empty and sealed aluminum sample pan was used as reference. Evaluation of the transition point was performed in the second heating step of two cooling/heating cycles (35°C to -65°C to 35°C with a scanning rate of 10°C/min and an equilibration duration of 1 min each) and furnished a glass transition temperature Tg' of -13.3°C, which corresponds to a theoretical maximal chamber pressure during sublimation phase of around 2 mbar.

The critical pressure for the sublimation phase was identified by critical pressure scanning (CPS) at moderate shelf temperature (10°C). Vials were filled with 5 ml of lyo solution and stoppers were placed in lyo-position. The vials were loaded onto the middle shelf of the freeze dryer. Vials were monitored by a macro video system. After freezing to -45°C/1000 µbar, the chamber pressure was set to the initial value of 50 µbar, shelf temperature was increased to the initial value (10°C) and lyophilization was allowed to run for one hour to generate a lyophilizate above the ice interface. Chamber pressure was then increased step wise (150, 250, 350, 500, 600, 800 µbar; 5 min ramp and 15 min constant pressure) and the beginning of collapse or other adverse effects was evaluated by macro photo monitoring. No macro-collapse was observed during the CPS. Freeze drying at high chamber pressure seemed to be possible for **IM-250** DP solution.

The DP solution was compounded as follows using 10R vials (Schott, Order-no.: 1470783) with 20 mm lyo stoppers, 4023, B2-TR, flurotec coating (West, Order-no.: 7002-5169):

| | |
|---|---|
| purified water | 3.5 g |
| HP-β-CD (150 mg/ml) | 0.75 g |
| **IM-250 HCl** (10 mg/ml **IM-250**) | 55 mg |
| 10% (w/w) Na₂HPO₄ (30 mM phosphate buffer) | 213 mg |
| adjust to pH 6.5 with 0.1 M HCl or 0.1 M NaHCO₃ | |
| adjust to final weight of 5.25 g with purified water | |
| **Final weight** | **5.25 g (5 ml)** |

Afterwards, the solution was passed over a 0.22 µm filter equipped with a PES membrane. 5 ml of this solution was filled into cleaned and depyrogenized 10R vials. After placing the autoclaved and dried stoppers into lyo position, the samples were loaded into the freeze dryer.

### Preparation:

HP-β-CD (467 g) was added in a beaker, then purified water (2189 g) was added and the suspension was stirred to homogeneous solution (pH was about 6.4). Then **IM-250 HCl** (34.2 g) was added and the mixture was stirred to homogeneity. The dissolution of **IM-250 HCl** within the HP-β-CD solution under stirring was rapid within minutes and resulted in a pH of about 1.9.

Then a 10% (w/w) Na₂HPO₄ solution (132.6 g) was added and the mixture was stirred to homogeneity, furnishing a clear solution. The pH was measured to give 6.36 and no pH adjustment was necessary. Purified water was added until a final weight of 3269 g was obtained.

This Example showed that the proper pH control and the order of addition is crucial for the manufacturing process. If the buffer is added before dissolving **IM-250 HCl,** a pH value of about 8.9 is set, which reduces the solubility of the API and leads to aggregations and precipitations which are not reversible even after prolonged stirring and making a filtration impossible.

The preferred freeze-drying process comprises loading the vial at 20°C, then initial freezing the vial at -5°C/1000 µbar for 1.5 h, followed by cooling to -45°C/1000 µbar for 6 h. Then the vacuum was adjusted to 200 µbar at this temperature for 30 min (ice condenser temperature -70°C). In a primary drying ramp the temperature was increased to 10°C at 200 µbar for 1 h, followed by the primary drying 10°C/200 µbar for 36 h. In the secondary drying ramp the temperature was increased to 35°C at 200 µbar during 2 h, followed by the secondary drying at 35°C/200 µbar for 5 h.

Primary drying duration with 200 µbar at 10°C shelf temperature was about 15 h, so the procedure contains a good safety margin. The macroscopic appearance of the lyophilizates shows no signs of cake defects. Some fogging was observed, which may be prevented by using different vial types (e.g. TopLyo vial). Reconstitution time by adding 4.5 ml of purified water was below 1 minute.

The analytical evaluation of the lyophilizate was as follows:

| | **n1/n2** |
|---|---|
| Residual moisture by Karl-Fischer [%] | 0.26/0.23 |
| Concentration of **IM-250** by RP-HPLC [mg/ml] | 10.29/10.31 |
| Purity of **IM-250** by RP-HPLC [%] | 99.18/97.91 |
| Turbidity [NTU] | 16.3/11.8 |
| pH | 6.67/6.68 |
| Particle analysis by Micro-flow imaging [counts/mL] | ≥10 µm: 153/264 |
| | ≥25 µm: 20/31 |

Acceptable results were obtained for RP-HPLC, residual moisture, pH and MFI.

An accelerated stability study was conducted at 2 to 8°C, 25°C and 40°C, respectively, for up to 6 months. Samples were taken and analyzed at time point (T = 0, 1, 3 and 6 months) with respect to visual appearance, documented by macro photography, reconstitution speed (lyo samples only), residual water content using a Karl-Fischer oven method, turbidity by nephelometry, osmolality (only at T = 0), micro-flow imaging, RP-HPLC for purity/content and pH.

No change in visual appearance was observed after 3 months storage at 2 to 8°C, 25°C and 40°C. Lyo samples were reconstituted by adding 4.5 mL purified water. Reconstitution behavior was satisfying with reconstitution times below 20 s under shaking. The obtained solution was clear and colorless. The analytical evaluation of the reconstituted lyophilizates in the accelerated stability study was as follows:

| | | | **T0** | **6 months 2-8°C** | **6 months 25°C** | **6 months 40°C** |
|---|---|---|---|---|---|---|
| Concentration of **IM-250** by RP-HPLC [mg/ml] | | n1 | 10.89 | 10.82 | 10.46 | 10.48 |
| | | n2 | 11.26 | 10.57 | 10.98 | 10.60 |
| Purity of **IM-250** by RP-HPLC [%] | | n1 | 98.97 | 98.70 | 98.74 | 98.77 |
| | | n2 | 99.03 | 98.69 | 98.94 | 98.89 |
| Turbidity [NTU] | | n1 | 5.38 | 5.07 | 5.18 | 7.05 |
| | | n2 | 5.39 | 5.13 | 6.62 | 7.61 |
| Residual moisture (KF) [%] | | n1 | 0.18 | 0.07 | 0.08 | 0.15 |
| | | n2 | 0.28 | 0.06 | 0.08 | 0.12 |
| MFI [counts/ml] | ≥10 µm | n1 | 462 | 262 | 126 | 234 |
| | | n2 | 319 | 246 | 169 | 300 |
| | ≥25 µm | n1 | 36 | 7 | 16 | 5 |
| | | n2 | 13 | 5 | 2 | 8 |

After 6 months storage at 2 to 8°C, 25°C and 40°C, the lyophilizates of **IM-250 HCl** are stable.

### Example 3: Rat PK Study to Investigate the Local Tolerance and Relevant Tissue Exposure of an Intravenous Administration of IM-250 HCl Formulations in Cyclodextrin Excipients in Comparison to Pritelivir

### Part A:

Male Sprague-Dawley (SD) rats arrived at an age of 7 weeks and were kept for an acclimatization period of 1 week. On day 0 of the study, the animals were assigned to the following groups (n = 6) and treated with the depicted formulations from Example 1 - Part C (all containing the same dose of 3 mg/kg **IM-250 HCl**):

| **#** | **Vehicle** | **Route** | **Volume** | **Sampling of blood** |
|---|---|---|---|---|
| A1 | 5% DMSO in 0.5% hydroxypropyl methylcellulose (HPMC) | p.o. | 3 ml/kg | 30, 60, 90, 240, 720, 1440 min |
| A2 | HP-β-CD 15 mg/mL from Example 1 | i.v. | 3 ml/kg | 5, 30, 90, 240, 720, 1440 min |
| A3 | SBE-β-CD 13.3 mg/mL from Example 1 | i.v. | 3 ml/kg | 5, 30, 90, 240, 720, 1440 min |
| A4 | 5% DMSO in heterologous rat serum | i.v. | 5 ml/kg | 5, 30, 90, 240, 720, 1440 min |

Intravenous administrations were performed under isoflurane anesthesia. The injection sites were marked to facilitate their later identification. Body weight was assessed before each treatment and rats were checked for any clinical signs associated with toxicity of the test substance. The rats were bled from the tail vein at the time points post administration as listed above. Blood samples were harvested in Na-heparinized tubes on ice. Subsequently, tubes were centrifuged at 6800 relative centrifugal force for 8 min at 4°C to generate plasma. Plasma samples were stored at -20°C until analysis by HPLC-MS/MS. The injection sites were checked for macroscopic signs of inflammation or toxicity at the following time points: 90 min post treatment (day 0), 4 h post treatment, 24 h post treatment, then once daily on days 1 to 3.

### Part B:

After a washout period of 3 days without treatment, the male Sprague-Dawley (SD) rats from Example 3 Part A were weighed and reorganized into new groups (n = 4) before treatment. Animals were treated with **IM-250 HCl** or Pritelivir free base (PTV) with the depicted formulations (all with the same dose of 3 mg/kg):

| **Group** | **Vehicle** | **Treatment** | **Route** | **Volume** |
|---|---|---|---|---|
| B1 | 5% DMSO in 0.5% HPMC | **IM-250 HCl** | p.o. | 3 ml/kg |
| B2 | HP-β-CD 15 mg/mL from Example 1 | **IM-250 HCl** | i.v. | 3 ml/kg |
| B3 | SBE-β-CD 13.3 mg/mL from Example 1 | **IM-250 HCl** | i.v. | 3 ml/kg |
| B4 | 5% DMSO in heterologous rat serum | **IM-250 HCl** | i.v. | 5 ml/kg |
| B5 | 5% DMSO in 0.5% HPMC | **PTV** | p.o. | 3 ml/kg |
| B6 | 5% DMSO in heterologous rat serum | **PTV** | i.v. | 5 ml/kg |

Intravenous administrations were performed under isoflurane anesthesia. Body weight was assessed before each treatment and rats were checked for any clinical signs associated with toxicity of the test substance. The injection sites were checked immediately before treatment and 4 h after. The rats were bled from the tail vein at 4 h post administration, right before euthanasia by i.p. administration of a lethal dose of pentobarbital (400 mg/kg in 5 ml/kg solution). To determine terminal compound concentrations, samples were taken from the brain (right cortex), spinal cord (section near the sacral ganglia), cerebrospinal fluid (CSF), trigeminal ganglia and sacral ganglia. Analytical samples were stored at -20°C until analysis by HPLC-MS/MS. Furthermore, skin from the injection site was sampled for histological analysis and transferred to 10% paraformaldehyde solution. After incubation in paraformaldehyde for 1 day, the skin samples were transferred into 70% ethanol.

### HPLC-MS/MS Measurements:

Test items (**IM-250 HCl** or **PTV**) were measured in plasma, CSF and extracted organ samples with an Agilent 1260 Infinity system coupled to a triple quadrupole Sciex API 4500 LC/MS/MS detector. A Dr. Maisch Reprosil-pur Phenyl column (50 x 2 mm, 3 µm) was used for the separation. The mobile phase was composed of water containing 0.1% formic acid (eluent A) and acetonitrile containing 0.1% formic acid (eluent B). Gradient used was: 90% A and 10% B for 1 min, to 100% B in 2 min, 100% B for 3 min, to 90% A and 10% B in 1 min and 90% A for 3 min. The standard curve of each compound (5-100000 nmol/L) and the quality controls (100, 1000 and 10000 nmol/L) were prepared by serial dilution in plasma. Precipitation of the proteins was achieved by adding 6 volumes of acetonitrile. The samples were homogenized by vortexing for 5 to 10 seconds and were then centrifuged for 10 min at 14000 rpm. Lower limit of quantification (LLOQ) of each test item was 46 nmol/L and upper limit of quantification (ULOQ) was 11111 nmol/L.

### Plasma, CSF and Organ Sample Preparation:

Six volumes of acetonitrile were added to the plasma or CSF samples. The samples were homogenized by vortexing for 5 to 10 seconds and were then centrifuged for 10 min at 14000 rpm. Organ samples were digested by adding one volume of proteinase K solution (0.5 mg/ml in 20 mmol/L sodium phosphate buffer) followed by incubation for 60 min at 50°C. Homogenates were generated by FastPrepTM tissue homogenization, two times at 6 m/s for 20 s each. Proteins were precipitated by adding 6 volumes of acetonitrile (+ internal standard) to the homogenate followed by another homogenization step at 6 m/s for 20 s. Samples were centrifuged for 10 min at 14000 rpm.

### Results - Part A:

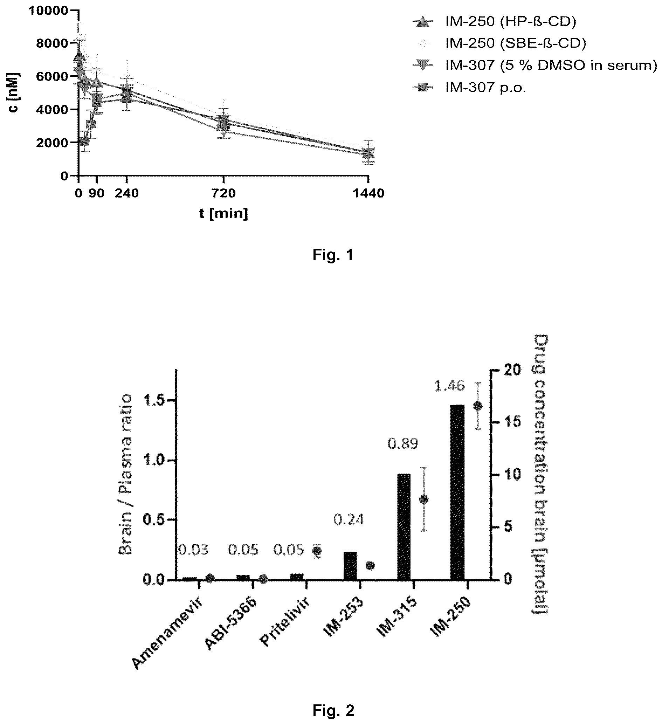

Body weight was stable over the course of the study and did not indicate any signs of toxicity or distress. No clinical signs of toxic effects were observed in any animal throughout the study and all animals survived their scheduled study period. The sites of injection in intravenously treated animals were checked at least once per day macroscopically by trained personnel. No signs of swelling, inflammation or other kinds of adverse reaction to the treatments were observed in any animal at any point during the study. The PK parameters (tail vein at various time points) are as follows (see Fig. 1 for PK curves):

| **Group** | | **Cₘₐₓ** | **AUC₀₋₂₄ₕ** | **elimination T_{1/2}** |
|---|---|---|---|---|
| A1 | **IM-250 HCl** (p.o.) | 4.6 µM | 75 µM*h | n.a. |
| A2 | **IM-250 HCl** in HP-β-CD (i.v.) | 6.2 µM | 83 µM*h | 10.8 h |
| A3 | **IM-250 HCl** in SBE-β-CD (i.v.) | 7.2 µM | 96 µM*h | 11.4 h |
| A4 | **IM-250 HCl** in serum (i.v.) | 8.3 µM | 73 µM*h | 10.9 h |

### Results - Part B:

The target exposures (in µM) 4 h post administration are as follows:

| **Group** | **plasma** | **CSF** | **spinal cord** | **brain** | **trigeminal ganglia** | **sacral ganglia** |
|---|---|---|---|---|---|---|
| B1 | 4.62 | 0.082 | 13.2 | 9.50 | 12.2 | 10.7 |
| B2 | 3.59 | 0.250 | 13.1 | 9.61 | 12.6 | 11.7 |
| B3 | 4.43 | 0.086 | 14.1 | 10.3 | 14.0 | 14.5 |
| B4 | 4.31 | 0.180 | 12.3 | 9.55 | 12.6 | 9.51 |
| B5 | 7.30 | 0.053 | 0.583 | 0.400 | 0.708 | 1.75 |
| B6 | 5.18 | 0.040 | 0.472 | 0.393 | 0.563 | 1.72 |

IM-250 shows a 2 to 3.6 fold higher concentration in the spinal cord, brain and trigeminal and sacral ganglia compared to plasma, while Pritelivir in contrast shows only a fraction (0.05 to 0.33) of the plasma concentration in the respective tissues. This results in a 17 to 22 fold higher ratio of IM-250/Pritelivir in the spinal cord, brain and trigeminal ganglia and in a 7 to 8 fold higher ratio in the sacral ganglia. Considering an IC₅₀ value for antiviral activity in vivo of 1 µM, IM-250 exceeds this threshold by at least an order of magnitude, while Pritelivir barely reaches this threshold to act on the helicase-primase target of herpes simplex viruses in the latent reservoir of neuronal tissues.

Regarding the CSF it can be noted that for **IM-250** the values are low compared to the values achieved in the spinal cord, brain, trigeminal ganglia and sacral ganglia, because in the CSF the free fraction and not the protein-bound values are measured. However, also the CSF values of IM-250 exceed the values achieved for Pritelivir.

### Conclusion:

Taken together, **IM-250 HCl** demonstrated long plasma stability in rats regardless of the formulation when given intravenously. Oral **IM-250 HCl** demonstrated excellent oral uptake, reaching similar plasma concentrations as the intravenous treatments from 90 min on (bioavailability of ~100%). The therapeutic target areas, namely the brain, spinal cord and ganglia, could be addressed by the compound in sufficiently high quantities and the measured concentrations indicated a preferential distribution compared to plasma. Furthermore, all intravenous treatments were tolerated well and there were no observances of skin irritation or otherwise adverse reaction around the injection sites at any point in the study.

### Example 4: Rat PK Study to Investigate the Tolerance of Multiple Doses via Intravenous Administration of IM-250 HCl Formulation in a Cyclodextrin Excipient

In a non-GLP study the PK of the lyophilized drug product **IM-250 HCl** for i.v. use (formulated with excipient HP-β-CD) was investigated after repeated i.v. administrations to male and female SD rats. In particular, the PK parameters after 14 days of once daily intravenous treatments of ~30 mg/kg **IM-250 HCl** were analyzed incl. determination of terminal exposure in the cortex and the drug product tolerability upon multiple doses was investigated.

A total of 6 male and 6 female SD rats arrived at an age of 7 weeks and were kept for an acclimatization period of 1 week. On day 0 of the study, the animals were bled from the tail vein to obtain baseline samples for hematology and clinical chemistry. From day 1 to day 14, once per day, the animals were treated with ~30 mg/kg of **IM-250 HCl** (in HP-β-CD from Example 2) or vehicle (HP-β-CD) (due to volume dilatation upon reconstitution of the lyophilized drug product in vials, the added volume of 5 ml increased to 5.4 ml reducing the final dose by a factor of 1.08 to 27.8 mg/kg **IM-250**). Treatments were applied intravenously with 3 ml/kg of a ~10 mg/ml **IM-250**/150 mg/ml HP-β-CD in 0.9% NaCl solution. Intravenous administrations were performed under isoflurane anesthesia, using a single-use 1 ml syringe with a 30 G needle at an application volume of 3 ml/kg. Injection locations on the tail were varied between treatments to reduce strain on the individual tissue.

Body weight was assessed on day 0 as well as once daily, before each treatment, and rats were checked for any clinical signs associated with toxicity of the test substance.

On days 1 and 14, the rats were bled from the tail vein at 5, 30, 60, 90, 240, 720 and 1440 min post administration. Blood samples were harvested in Na-heparinized tubes, on ice. Subsequently, tubes were centrifuged at 6800 rcf for 8 min at 4°C to generate plasma. Plasma samples were stored at -20°C until analysis. For subsequent analysis of heart plasma, kidney, liver and cortex, animals were euthanized at 24 h after last dosing (last dosing day 14, termination day 15) and samples of the respective organ tissues were taken. Samples for HPLC-MS/MS measurements were stored at -20°C until analysis. On day 15, 24 h after the last treatment on day 14, the animals were sacrificed by inhalation of CO₂ and cardiac puncture. Heart blood, plasma and organ samples were taken for subsequent analysis. Samples for histological analysis were incubated in 4% paraformaldehyde solution for 24 h at rt and then transferred to 70% ethanol. Organ samples were digested by adding one volume of proteinase K solution (0.5 mg/ml in 20 mmol/L sodium phosphate buffer) followed by incubation for 60 min at 50°C. Homogenates were generated by FastPrep^{™} tissue homogenization, two times at 6 m/s for 20 s each. Proteins were precipitated by adding 6 volumes of acetonitrile (+ internal standard) to the homogenate followed by another homogenization step at 6 m/s for 20 s. Samples were centrifuged for 10 min at 14000 rpm.

### Results:

Body weight was stable over the course of the study. No other clinical signs or toxic effects were observed in any animal throughout the study and all animals survived their scheduled study period. The sites of injection in intravenously treated animals were checked at least once per day macroscopically by trained personnel. No signs of swelling, inflammation or other kinds of adverse reaction to the treatments were observed in any animal at any point during the study. For all animals of the study, no macroscopic findings were reported. No irregularities or adverse skin reactions were observed around the point of injection and there were no abnormalities observed upon necropsy. Spleen weights of rats treated with **IM-250** were minimally lower than those of control the animals at the end of the study (from 0.25±0.02% to 0.22±0.01% of body weight for male animals and 0.3±0.05% to 0.28±0.03% for female animals), but the differences were not statistically significant (T-test). The PK parameters are as follows:

| **Group** | | **day 1 AUC₀₋₂₄ₕ** | **elimination T_{1/2}** | **day 14 AUC₀₋₂₄ₕ** |
|---|---|---|---|---|
| 1 | **IM-250** (male) | 587 µM*h | 8.2 h | 577 µM*h |
| 2 | **IM-250** (female) | 994 µM*h | 15.6 h | 1007 µM*h |

On day 15, 24 h after the last treatment, the rats were euthanized by inhalation of CO₂ and cardiac puncture and heart plasma, cortex, liver and kidney tissues were sampled. Terminal heart plasma and cortex concentrations are as follows:

| **Group** | | **plasma** | **cortex** |
|---|---|---|---|
| 1 | **IM-250** (male) | 8.1±3.1 µM | 17.6±3.8 µM |
| 2 | **IM-250** (female) | 16.7±5.0 µM | 44.5±14.6 µM |

### Conclusion:

Repeated intravenous treatments of **IM-250** over 14 days were tolerated well, with negligible reduction of body weight versus control animals and no relevant clinical, hematological or macroscopic signs of toxicity. Furthermore, there were no observances of skin irritation or otherwise adverse reaction around the injection sites at any point in the study. Treatments were similarly well tolerated between male and female specimens, despite female rats displaying higher terminal plasma and cortex levels of **IM-250** and increased AUC₀₋₂₄ₕ. Any observed hematological of clinical chemistry changes were consistent with those in the vehicle groups and are likely to be related to treatment with the vehicle's excipient, HP-β-CD.

A GLP study confirmed this results. Once daily intravenous injection of **IM-250** at doses up to 30 mg/kg/day for 2 consecutive weeks was well tolerated in Sprague-Dawley rats. The lyophilisized HP-β-CD formulation according Example 2 was used. Test article-related findings included lower body weight gains and food consumption in females administered ≥10 mg/kg/day, alternations in clinical chemistry and hematology parameters in animals administered ≥10 mg/kg/day, decreases in thymus weights in animals administered ≥10 mg/kg/day. Findings were partially or completely recovered by the end of the 2-week recovery period and none of these alterations are considered adverse. The no-observed-adverse-effect-level (NOAEL) is determined to be 30 mg/kg/day in this study, corresponding to Cₘₐₓ of 69 and 94 µM and AUCₗₐₛₜ of 351 and 873 µM*hr in males and females, respectively, on Day 14.

### Example 5: Manufacturing IM-250 50 mg - Powder for Solution for Infusion (Sterile Powder) in Compliance to GMP Regulations

### Description and dosage form

**Product: IM-250** 50 mg / powder for solution for infusion
**Dosage form:** Sterile, lyophilized powder

### Composition

One vial **IM-250** 50 mg drug product contains:

| | |
|---|---|
| • **IM-250** HCl* Ph. Eur./ internal spec. | 54.2 mg** |
| • 2-Hydroxypropyl-beta-cyclodextrin (HP-β-CD) Ph. Eur. | 750 mg |
| • Na₂HPO₄•12 H₂O for a 10% (w/w) aqueous solution Ph. Eur. | 537.36 mg |
| • Hydrochloric acid 37% (used as 0.1 mol/kg solution) Ph. Eur. | q.s. to pH 6.5 as required |
| • Sodium bicarbonate (used as 0.1 mol/kg solution) Ph. Eur. | q.s. to pH 6.5 as required |

| | |
|---|---|
| ** corrected according to the assay (w/w) of the respective drug substance batch | |

### Primary packaging material

- injection vial 10R, clear glass, Fiolax, Schott
- lyo stopper, 20 mm, bromobutyl rubber, 1319 4023/50, grey, West
- flip off cap, 20 mm, FOS 5115, red PP disc, West

### Production

Sterilization of material and equipment that come into contact with product is done as per standard operation procedure (e.g. at not lower than 121°C and 20 min). Weighing the raw material is done under laminar flow in clean room class C with appropriate protection for personnel as well as for product. A sterile environment is maintained throughout the production.

### Preparation of the compounding solution

Water for injections (66 % of final weight) is added ca. 20°C to the compounding tank. The amount of HP-β-CD is added while stirring (ca. 250 rpm). The solution is stirred until complete dissolution (at least 10 min). The amount of **IM-250 HCl** is added while stirring (ca. 250 rpm) and the solution is stirred until complete dissolution (at least 10 min). Subsequently, the 10% Na₂HPO₄•12 H₂O solution is added while stirring. The pH is measured and, if necessary, adjusted with a 0.1 mol/kg HCl solution or 0.1 mol/kg NaHCO₃ solution to a value of 6.3 to 6.7 (target: 6.5). The solution is stirred until complete dissolution (at least 10 min) and the pH is measured again. The solution is filled up with water for injection at 15 to 25°C to the final weight. The solution is filtered through two sterilized membrane filters (0.2 µm pore size; 1^{st} filter: bioburden reduction filter (e.g.Kleenpak Fluorodyne II), 2^{nd} filter: sterile filter) under nitrogen pressure (approx. 1.5 bar overpressure).

### Filling, stoppering and freeze-drying

Filling into sterile glass vials is performed under laminar flow inside a Restricted Access Barrier System (RABS) in clean room class A. Filled amount per vial is 5.00 ml (5.25 g). During filling, the weight of the filled amount per vial is checked automatically on a random basis to check filling precision. After filling, previously sterilized stoppers are partially inserted into the filled vials automatically so that freeze-drying can be performed. The vials are automatically loaded into the pre-sterilized freeze-dryer. After the lyophilisation program is completed the stopper is pressed on its final position by the freeeze dryer's hydraulic system. The pressure in the freeze dryer is at 750 mbar during final closure of the vials. The freeze-drying is controlled by use of a specific recipe entered into a Programable Logic Controller (PLC). The duration of the freeze-drying cycle is 44 hours and 40 min according to the following scheme:

| **No** | **Step** | **temp. [°C]** | **pressure [mbar]** | **time [hh:mm]** |
|---|---|---|---|---|
| 1 | Loading | +20 | 1000 | 00:10 |
| 2 | Freezing | +20 to -5 | 1000 | 00:30 |
| 3 | Freezing | -5 | 1000 | 01:00 |
| 4 | Freezing | -5 to -45 | 1000 | 01:00 |
| 5 | Freezing | -45 | 1000 | 05:00 |
| 6 | Vacuum Adjustment | -45 | 0.2 | 00:30 |
| 7 | Primary drying ramp | -45 to +10 | 0.2 | 01:30 |
| 8 | Primary drying | +10 | 0.2 | 28:00 |
| 9 | Secondary drying ramp | +10 to +35 | 0.2 | 02:00 |
| 10 | Secondary drying | +35 | 0.2 | 05:00 |

Capping is performed according to standard operation procedure with push off caps with red plastic disc. The filled and closed product is stored at 15 to 25°C.

### Example 6: Phase 1 Clinical Trial to Evaluate the Safety, Tolerability, and Pharmacokinetics of Single and Multiple Intravenous Ascending Doses of IM-250 and Drug Interaction with Intravenous Acyclovir in Healthy Volunteers

### Primary objective

To evaluate the safety and tolerability of single and multiple intravenous ascending doses of **IM-250** in healthy volunteers.

### Secondary objective

To define the dose range for subsequent trials investigating **IM-250** intravenous administration for herpes encephalitis treatment.

### Primary endpoints

Occurrence (number) of dose-limiting toxicities (DLT)
- Serious adverse reaction (i.e., a serious adverse event (SAE) considered at least possibly related to the study medication administration) during the exposure and within 28 d after the end of exposure.
- Severe (Common Terminology Criteria for Adverse Events (CTCAE) grade III) non-serious adverse reactions (i.e., severe non-serious adverse event (AE) considered as, at least, possibly related to the study medication administration) lasting more than 72 h during the exposure and within 28 d after the end of exposure.

### Secondary endpoints

Non-compartmental pharmacokinetic (PK) analysis of **IM-250** plasma concentrations, more precisely:
Exposure with different intravenous doses of **IM-250** as defined by
- the area under the plasma concentration-time curve (AUC₂₄, AUC_{0-∞}),
- maximum and minimum plasma concentration (Cₘₐₓ, Cₘᵢₙ).

### Further secondary objectives

- To describe the PK after single intravenous doses of **IM-250**
- To describe the PK after multiple intravenous doses of **IM-250**
- To describe the safety profile of single intravenous doses of **IM-250**
- To describe the safety profile of multiple intravenous doses of **IM-250**

### Further secondary endpoints

Further description of the non-compartmental PK analysis of IM-250 plasma concentrations, i.e., description of the following PK parameters:
- time to reach Cₘₐₓ (Tₘₐₓ),
- plasma concentration at 24 h (C₂₄ₕ), 5 d (C_{5d}), and 8 d (C_{8d})
- half-life (t_{1/2}),
- apparent clearance (CL/F),
- mean residence time (MRT),
- volume of distribution (V_{d}/F), and
- T>C_{target}.

Further derived parameters may be calculated, if deemed necessary.

### Safety secondary endpoints

- Description of all AE and treatment-emerging AE, i.e.
- System organ class (SOCs),
- Seriousness,
- Relatedness,
- Severity, and
- Outcome.

### Study Medication

50 mg powder for reconstitution for infusion in vials reconstituted with water for injection prior to administration. The study medication is then applied as intravenous infusion in 250 ml of 0.9% isotonic solution of sodium chloride. The reconstitution scheme for each cohort is provided in the study protocol.

### Study Design

The study consists of two parts investigating single intravenous (i.v.) (SAD) and multiple intravenous (MAD) doses of **IM-250** in healthy volunteers. As part of the SAD study, drug interaction between **IM-250** and intravenous Acyclovir (ACV) is investigated.

### SAD study

single intravenous ascending dose study consists of four consecutive cohorts:
Cohort 1: 6 subjects, **IM-250,** 25 mg, single i.v. dose
Cohort 2: 6 subjects, **IM-250,** 50 mg, single i.v. dose
Cohort 3: 6 subjects, **IM-250,** 100 mg, single i.v. dose
Cohort 4: 6 subjects, **IM-250,** 200 mg, single i.v. dose
   Two additional cohorts investigate the DDI potential with Acyclovir and are conducted upon data availability of 100 mg single dose cohort.
Cohort 5: 6 subjects, **IM-250,** 100 mg, single i.v. dose + ACV, 10 mg/kg, single i.v. dose
Cohort 6: 6 subjects, ACV, 10 mg/kg, single i.v. dose

Participants receive **IM-250,** ACV or combination as a single intravenous dose. After all subjects of cohorts 1-3 are exposed and complete the visit Day 7, an independent data and safety monitoring board (DSMB) reviews the relevant safety data in a timely manner and provides advice on the continuation, modification, or termination of the SAD study. Given a positive DSMB decision, the next dose level cohort is started.

Cohorts 4, 5 and 6 start in parallel upon completion cohort 3 and a positive DSMB decision.

### MAD study

A multiple intravenous ascending dose study is started upon completion of the cohort 1 Day 7 of the SAD study and a positive DSMB decision, and consists of three consecutive cohorts:
Cohort 7: 6 subjects, **IM-250,** 25 mg, multiple i.v. doses, 14 days
Cohort 8: 6 subjects, **IM-250,** 50 mg, multiple i.v. doses, 14 days
Cohort 9: 6 subjects, **IM-250,** 100 mg, multiple i.v. doses, 14 days
Subjects receive multiple once daily doses of **IM-250** intravenously for 14 days. After all participants of a MAD cohort complete the visit Day 21 (7 days after completion of the exposure) an independent DSMB reviews the relevant safety data in a timely manner and provide advice on the continuation, modification, or termination of the MAD study. Given a positive DSMB decision, the next dose level cohort starts.

### Study procedures

### Screening period:

During the screening period (within 28 d prior to the first day of dosing), the eligibility of participants is confirmed. Eligibility is re-confirmed on admission to the trial center (Day 1).

### Inpatient period

Participants remain in the study site:
- from Day -1 until Day 2 (24 h post-administration) in SAD study
- from Day -1 until Day 2 and at Day 14 (hospitalization 1 day before) for 24 hours in MAD study.

### Treatment

**IM-250** is administered intravenously as a single dose in the SAD cohorts and once per day for 14 days in the MAD cohorts.

During the MAD study the participants visit the study site every day for i.v. IM-250 administration and undergo physical examinations, blood and urine sampling for safety laboratory testing, electrocardiogram, vital signs, adverse events, and concomitant medication collection at the appropriate days.

PK samples are collected before the study medication administration and afterwards at defined time intervals:
- For SAD study during the inpatient period: pre-dose, 0.25, 0.5, 0.75, 1, 1.25, 1.5, 1.75, 2, 2.5, 3, 3.5, 4, 5, 6, 8, 10, 12, 14, 18, 24. And on further visits: 48 h, 72 h, days 3, 4, 5, 8, 14, 28 (EOS).
- For MAD study:
   - Profile at Day 1: pre-dose, 0.25, 0.5, 0.75, 1, 1.25, 1.5, 1.75, 2, 2.5, 3, 3.5, 4, 5, 6, 8, 10, 12, 14, 18, 24
   - Profile at Day 14: pre-dose, 0.25, 0.5, 0.75, 1, 1.25, 1.5, 1.75, 2, 2.5, 3, 3.5, 4, 5, 6, 8, 10, 12, 14, 18, 24

And at the following study days: 3, 4, 5, 8, 14, 21, 28, 42 (EOS).

### Follow up

During the follow-up period for 28 days, the participants visit the study center:
- In SAD cohorts: at days 3, 4, 5, 8, 14 and 28 (EOS);
- In MAD cohorts: at days 21, 28, 42 (EOS).

All subjects are requested to use highly effective contraception during the study.

### Example 7: The Adaptive Design, Double-Blind, Randomized, Placebo-Controlled Phase 2/3 Clinical Trial to Evaluate Efficacy and Safety of IM-250 in Patients with Herpes Encephalitis.

### Primary objective

Determination if **IM-250** can improve outcome in patients with herpes encephalitis if given intravenously on top of the standard therapy.

### Secondary objective

Definition of safety and tolerability profile of intravenous **IM-250** in patients with herpes encephalitis.

### Primary endpoint

Change to baseline in Clinical Global Impression (CGI) severity of illness scale at 180 days after first study medication administration.

### Secondary efficacy endpoints

- Repeatable Battery for the Assessment of Neuropsychological Status (RBANS) at 90 days, and 180 days
- HSV PCR-positivity in CSF on Study Day 4
- Seizure activity (clinical, EEG) at 30 days, 90 days, and 180 days
- Mortality at 30 days, 90 days, 180 days, and 1 year

### Safety endpoints

- Incidence and severity of adverse events
- Laboratory abnormalities
- Physical examination and vital signs
- 12 lead ECG

### Study duration

- Screening period: up to 12 hours
- Treatment period: 21 days
- Follow up period: 49 weeks
- Total duration of the study: 1 year

### Study Design

### Mode of Administration

Acyclovir is administered for 21 days as intravenous infusion 3 times daily at a dose of 10 mg/kg.

**IM-250** is administered as intravenous infusion once per day for 14 days. A single loading dose is given in **IM-250** group on the first day of treatment, followed by 13 days of administration of maintenance dose. The **IM-250** dose is determined upon availability of data from Phase 1 clinical trial with intravenous formulation.

### Study groups

- Group 1: **IM-250** + Acyclovir
- Group 2: Placebo + Acyclovir

### Study procedures

### Screening period

During the screening period (within 12 h prior to the first dosing), the eligibility of patients is confirmed.

### Randomization and treatment period

Study medication is administered in-patient. During the treatment period patients undergo daily assessments.

### Follow up

During the follow-up for 49 weeks, patients visit the Study Center at Day 30, Day 90, Day 180, Day 365 (EOS).

All patients are requested to use highly effective contraception for 2 months after the last study drug administration.

### Example 8 - Post Administration Exposure of Different Helicase Primase Inhibitors

The present Example describes the evaluation of post administration exposure of a variety of helicase primase inhibitors in plasma, blood, organs and nervous system, in particular in ganglia and brain. The comparative evaluation shows that after single or multiple doses of helicase primase drugs only concentrations of the helicase-primase inhibitor according to the present invention (Compound of the Formula (I); IM-250) reach high levels in the nervous system that are in the range of plasma or blood levels (ratio 0.5-4 of plasma or blood to nervous system) in diverse animal species, while other helicase primase inhibitors, e.g. Amenamevir, Pritelivir or ABI-5366, show a low brain to plasma ratio of less than 0.1. In preclinical animal studies performed so far, treatment with the blood brain barrier and blood nerve penetrating **IM-250** reduced the reactivating competence of the neuronal latent herpes viral reservoir.

A variety of structurally diverse helicase primase inhibitors (HPIs listed in Table 2 below) have been synthesized and administered to animals at varying doses and frequency and the pharmacokinetic profile was analyzed with respect to exposure in plasma, blood and organ tissue, in particular the brain and ganglia, at discrete times points post administration.

The blood, plasma and brain exposure of diverse HPIs was measured after a single administration of the respective helicase primase inhibitors in mice at 10 mg/kg. Surprisingly, a structural relationship of helicase primase inhibitors with respect to brain exposure ranking from low to high was observed (Table 2, ABI-5366 < Amenamevir < IM-201 < IM-253 < IM-205 < Pritelivir < IM-315 < IM-204 and < IM-250 (Adibelivir)) and the corresponding blood or plasma to brain ratio increases in the following order (Table 2 and Fig. 2, Amenamevir < ABI-5366 < Pritelivir < IM-201 < IM-253 < IM-205 < IM-315 < IM204 and < IM-250 (Adibelivir). A low brain/plasma ratio of < 0.1 was observed for Amenamevir, ABI-5366 and Pritelivir, a medium ratio of 0.2 to 0.5 was calculated for IM-201, IM-253 and IM-205 and a high ratio of brain/plasma exposure of 0.5 to 1.5 is shown for IM-315, IM-204 and IM-250. IM-250 showed the highest brain/plasma ratio and the highest drug concentration in the brain. The plasma/brain ratio (range 1.1 to 1.5) is relatively constant with respect to the dose and frequency administered in the range of 10-300 mg/kg as demonstrated for IM-250 and IM-253 (0.2-0.3, Table 2). The ratios of higher brain exposure follow a trend or function of combined increasing AlogP and decreasing polar surface areas (TPSA) as depicted in Table 2.

Finally, the blood/plasma ratio of IM-250 in C57BL/6J male mice is 1.0 (at 10 and 100 mg/kg per os at 24 hours post administration).

Table 2 shows the exposure of helicase primase inhibitors (HPIs) in blood or plasma and brain of mice (C57BL/6J) at discrete time points (2-24 hours) after administration of single or multiple doses of 10-300 mg/kg in 0.5% HPMC/PBS 7.4:

**Table 2**

| Exposure of helicase primase inhibitors (HPls) in blood or plasma and brain of mice (C57BL/6J) at discrete time points (2-24 hours) after oral administration of single or multiple doses of 10-300 mg/kg in 0.5% HPMC/PBS 7.4. | | | | | | | | |
|---|---|---|---|---|---|---|---|---|
| **Compound** | **tPSA** ^{(a)} [A²] **AlogP** | **Dose** (mg/kg) per os | **Cmax** [µM] | **AUC₂₄** (ng*h/ml) | **T_{1/2}** (hrs) | **Plasma** or **Blood** (µM) | **Brain** (µmolal) (hrs) | **Ratio** Brain/ plasma |
| IM-205 | 100 | **10 SD** | **13.9** | **37600** | **3.4** | **5.6** Blood | **2.3 (6h)** | **0.4** |
| 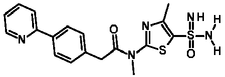 | | | | | | | | |
| | 3.2 | | | | | | | |
| IM-204 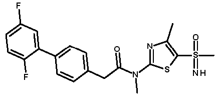 | 74 | **10 SD** | **15** | **59997** | **6.9** | **11.1** | **14 (4h)** | **1.3** |
| | 4.1 | | | | | Blood | | |
| IM-250 (adibelivir) | 74 | **10 SD** | **19** | **85634** | **6-7** | **11.4** | **17 (4h)** | **1.5** |
| 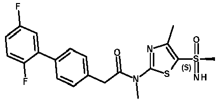 | | 30 SD | | | | 18 | 25 (4h) | 1.4 |
| | | 100 SD | | | | 54 | 81 (4h) | 1.5 |
| | | 300 SD | | | | 56 | 64 (4h) | 1.1 |
| | 4.1 | 100 SD | | | | 64 | 96 (4h) | 1.5 |
| | | 100 MD | | | | 95 | 146 (4h) | 1.5 |
| | | Day 7 | | | | | | |
| IM-315 | 93 | **10 SD** | **9.3** | **75613** | **^{~}20** | **8.6** | **7.7 (4h)** | **0.9** |
| 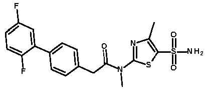 | | | | | | | | |
| | 2.9 | | | | | | | |
| IM-201 | 86 | **10 SD** | **11** | **22464** | **2.3** | **5** | **0.8 (4h)** | **0.2** |
| 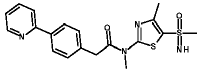 | | | | | | | | |
| | 2.9 | | | | | Blood | | |
| IM-253 | 86 | **10 SD** | **17** | **38023** | **1.9** | **5.8** | **1.4 (4h)** | **0.2** |
| 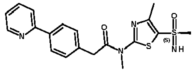 | | 300 MD | | | | 106 | 29 (2h) | 0.3 |
| | 2.9 | Day 4 | | | | | | |
| Pritelivir | 105 | **10 SD** | **37** | **186000** | **6** | **55** | **2.8 (4h)** | **0.05** |
| 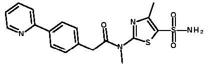 | | | | | | | | |
| | 1.8 | | | | | | | |
| Amenamevir | 118 | **10 SD** | **5.8** | **23500** | **2.4** | **7.2** | **0.2 (4h)** | **0.03** |
| 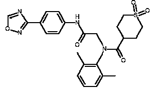 | | | | | | | | |
| | 2.2 | | | | | 7 | 0.3 (2h) | 0.05 |
| ABI-5366 | 96 | **10 SD** | **7.3** | **61806** | **~20** | **3.1** | **0.1 (4h)** | **0.05** |
| 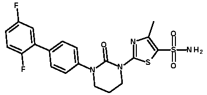 | | | | | | | | |
| | 2.9 | | | | | 3.2 | 0.2(24h) | 0.06 |

| | | | | | | | | |
|---|---|---|---|---|---|---|---|---|
| **(a)** tPSA values were calculated with ChemDraw 22.0 and AlogP (Ghose-Crippen-Viswanadhan-Octanol-Water-distribution coefficient with BIOVIA Draw 2022 | | | | | | | | |

In another study the brain/plasma ratio of IM-250 in diverse species at defined time points ranging from 4 to 24 hours after administering diverse doses of 0.5-300 mg/kg orally or intravenously either as single (SD) or multiple doses (MD) over several, up to 28 days, was analyzed.

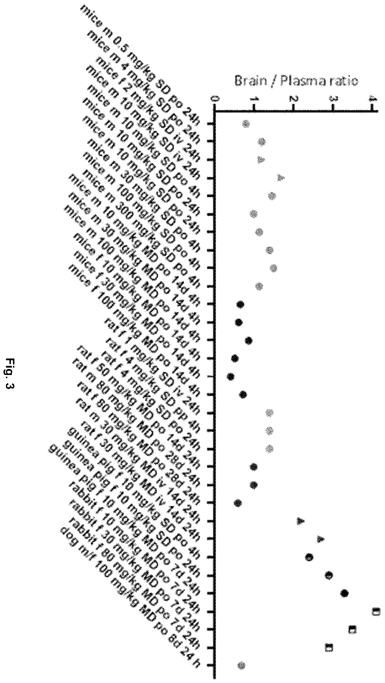

The brain/plasma ratio is constant in the range of 0.4 to 1.5 in mice, rats and dogs after giving single and multiple doses of helicase primase inhibitors analyzed at diverse time points post administration. The ratio is higher for guinea pigs and rabbits with a range of 2.4-3.4 and 2.9-4.1, respectively. The iv administration of 30 mg/kg to rats formulated in hydroxypropyl-β-cyclodextrin (HP-β-CD) shows slightly higher ratios in the range of 2.2-2.7 as compared to oral administrations. The brain/plasma ratio appears to be constant independent of the gender of species treated as show e.g. for male and female rats (see Fig. 3).

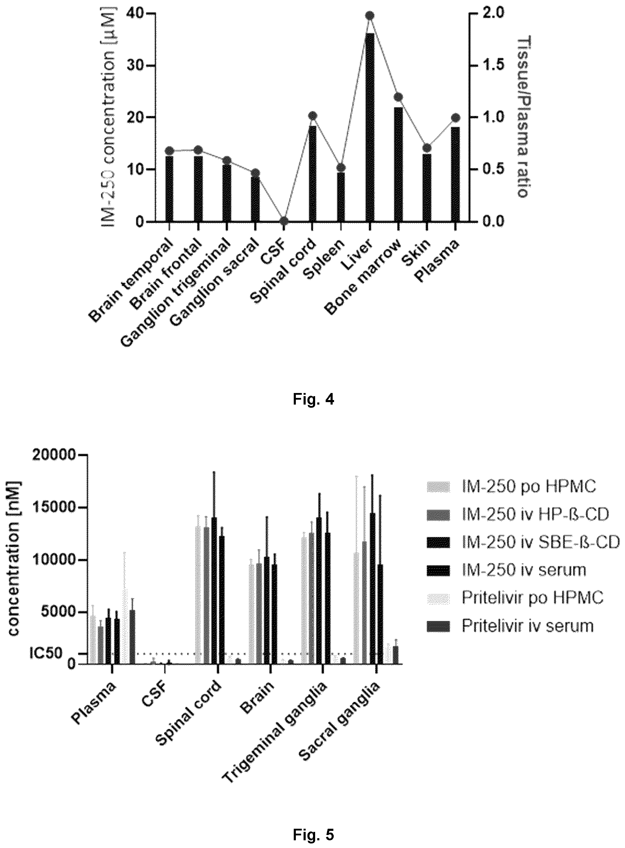

Next, the permeability of IM-250 (Adibelivir) was analyzed with respect to the tissue distribution as well as blood brain and blood nerve barrier in non-rodents (dogs). The overall tissue distribution of IM-250 was analyzed in beagle dogs after multiple once daily doses of 100 mg/kg for 8 consecutive days at 24 hours post last administration (see Fig. 4). IM-250 is equally distributed over the brain, ganglia, spinal cord, bone marrow, spleen and skin within a tissue/plasma ratio of 0.5 to 1.2 at a terminal plasma concentration of 18.2 µM. Liver samples show a higher and the cerebrospinal fluid (CSF) a lower exposure than this narrow range of 0.5 to 1.2. IM-250 is mainly excreted via the bile; this may explain the higher ratio of 2 for liver tissue. Due to its high protein binding of ~99%, the CSF/plasma ratio is 0.01, because the CSF is low in protein concentration and the CSF thereby mimics somehow the free fraction of IM-250 in the CSF (~100 nM) while the total concentration in other tissues is roughly 8-22 µM.

Finally, the permeability of iM-250 (Adibelivir) and Pritelivir was analyzed with respect to the blood brain and blood nerve barrier in rodents (SD rats). Single doses of 3 mg/kg of Adibelivir, IM250 HCl salt and Pritelivir were formulated for oral administration and intravenous injections and administered orally or via slow bolus injection to SD rats. 4 hours post administration the drug concentrations were analyzed in plasma, CSF, spinal cord, brain and trigeminal and sacral ganglia. Irrespective of the formulation or administration route the exposure of Adibelivir and Pritelivir in plasma is in the range of 3.5-4.6 and 5.2-7.3 µM, respectively. While Adibelivir shows high exposure in the spinal cord, brain and trigeminal and sacral ganglia exceeding the *in vivo* IC₅₀ of ~1 µM (accounting for the protein binding) at least 10 times, Pritelivir in contrast barely reaches the in vivo IC₅₀.

Again, while the ratio of brain/plasma was 0.05 and 0.08 for Pritelivir, the ratio of IM-250 was 2.1 after oral gavage and up to 2.6 after slow bolus iv injections, respectively. As observed also in non-rodents (beagle dogs), here in rodents, the concentration of Adibelivir (82 -250 nM) and Pritelivir (40-53 nM) in the CSF is low and, as outlined above, due to the low protein concentration in the CSF in contrast to the surrounding tissues and plasma thereby mimicking somehow the free fraction of the drug analyzed in the CSF (see Fig. 5).

### Conclusion:

The results of this Example show that IM-250 has exposure in brain and ganglia in the range of plasma concentration, whereas other HPIs such as Amenamevir, Pritelivir and ABI-5366, show only an exposure of less than 10% in the nervous system compared to plasma levels.

The brain/plasma ratio is roughly constant over time with respect to dose, formulation, frequency and route of dosing and species or gender.

## Claims

1. A pharmaceutical composition comprising a compound according to Formula (**I**) 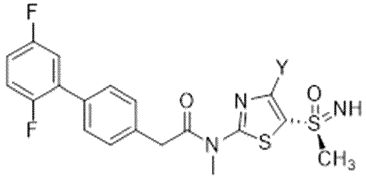
wherein Y is selected from CH₃ and CD₃;
or a pharmaceutically acceptable salt, co-crystal, hydrate or solvate thereof,
and one or more cyclodextrins.

2. The pharmaceutical composition according to claim 1, which is a solid composition, preferably a lyophilizate.

3. The pharmaceutical composition according to claim 1, which further comprises a pharmaceutically acceptable aqueous solution, preferably a physiological solution, more preferably an intravenous injection solution or an infusion solution.

4. The pharmaceutical composition according to any one of the preceding claims for the use in parenteral administration, preferably for the use in intravenous administration or for infusion.

5. The pharmaceutical composition according to any one of the preceding claims comprising the compound of the Formula (I), or its isotopic variants, pharmaceutically acceptable salts, co-crystals, hydrates or solvates, in a dose strength of 10 to 600 mg per single dose;
preferably in a dose strength of 10, 20, 25, 50, 100, 200 or 400 mg per single dose; more preferably in a dose strength of 50, 100, 200 or 400 mg per single dose;
even more preferably in a dose strength of 200 or 400 mg per single dose;
calculated based on the weight of Formula (I) as the free base.

6. The pharmaceutical composition according to any one of the preceding claims, comprising a cyclodextrin selected from 2-hydroxypropyl-β-cyclodextrin (HP-β-cyclodextrin / HP-β-CD) and/or sodium sulfobutylether-β-cyclodextrin (SBE-β-cyclodextrin / SBE-β-CD), preferably comprising the cyclodextrin HP-β-cyclodextrin.

7. The pharmaceutical composition according to any one of the preceding claims, wherein the compound of the Formula (I) is selected from the group: 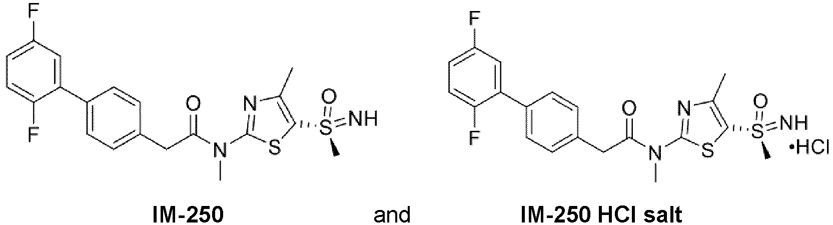
or a co-crystal, hydrate or solvate thereof,
preferably the compound of the Formula (I) is the HCl salt **IM-250 HCl**;
more preferably the compound of the Formula (I) is selected from crystalline forms of the **IM-250 HCl salt characterized by** an X-ray powder diffractogram comprising at least 4 of the following peaks (±0.2 degrees 2θ): 13.7, 17.0, 17.7, 19.8, 21.8 and 22.8, as determined on a diffractometer using Cu-Kα radiation at a wavelength of 1.54 Å.

8. The pharmaceutical composition according to any one of the preceding claims, further comprising
(i) one or more pharmaceutically acceptable carrier; and/or
(ii) one or more excipients, such as a buffer and/or a tonicity adjusting agent, preferably a buffer selected from Na₂HPO₄ and a hydrate thereof, more preferably Na₂HPO₄•12 H₂O, and/or a tonicity adjusting agent selected from glucose and sodium chloride, more particularly sodium chloride; and/or
(iii) one or more additional active substances being effective in treating a disease or disorder associated with viral infections (antiviral active compounds), preferably an additional active substance being effective in treating herpes infections; even more preferably Acyclovir.

9. The pharmaceutical composition according to any one of the preceding claims, comprising the compound of the Formula (I) in the form of the free base IM-250 or its HCl salt 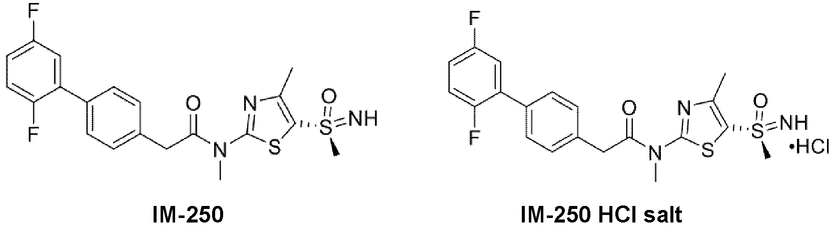
a cyclodextrin selected from HP-β-cyclodextrin and SBE-β-cyclodextrin,
a buffer, preferably Na₂HPO₄ or a hydrate thereof, more preferably Na₂HPO₄•12 H₂O, and
a pharmaceutically acceptable aqueous solution, preferably a physiological solution.

10. The pharmaceutical composition according to any one of the claims 1 to 9
• for the use in the prophylaxis and treatment of a disease or disorder associated with viral infections caused by herpes viruses, such as in particular by herpes simplex viruses and/or
• for the use in treating and reducing or eliminating latent (dormant) forms of herpes viruses in neuronal tissue, nerves and meninges, preferably for avoiding or preventing recurrence and reactivation of herpes infections or even severe implications associated therewith, such as Mollaret's meningitis or herpes simplex encephalitis (HSE), and/or
• for the use in the prophylaxis and treatment of neurodegenerative diseases caused by viruses, including Alzheimer's disease caused by viruses, such as caused by Herpes simplex viruses, and/or
• for the use in the prophylaxis and treatment of diseases caused by or associated with Herpes simplex infections, which are selected from encephalitis, neonatal herpes esophagitis and Alzheimer's disease.

11. The pharmaceutical composition according to any one of the claims 1 to 9 for the use according to claim 10 for the prophylaxis and treatment of patients displaying Herpes labialis, Herpes genitalis and Herpes-related keratitis, Herpetic esophagitis, Herpetic pneumonia, Alzheimer's disease, encephalitis, Mollaret's meningitis, pneumonia, hepatitis; patients with a suppressed immune system, such as AIDS patients, cancer patients, patients having a genetic immunodeficiency, transplant patients; new-born children and infants; Herpes-positive patients, in particular Herpes-simplex-positive patients, for suppressing recurrence or viral shedding (suppression therapy); patients, in particular in Herpes-positive patients, in particular Herpes-simplex-positive patients, who are resistant to nucleosidic antiviral therapy such as Acyclovir, Penciclovir, Famciclovir, Ganciclovir, Valacyclovir and/or compounds such as the phosphonates Foscarnet or Cidofovir.

12. The pharmaceutical composition for the use according to claim 10 or 11, comprising the parenteral administration, preferably intravenous administration or infusion, of the composition with a daily dose amount of 10 to 600 mg of the compound of Formula (I), or its isotopic variants, pharmaceutically acceptable salts, co-crystals, hydrates or solvates;
preferably with a daily dose amount of 10, 20, 25, 50, 100, 200 or 400 mg;
more preferably with a daily dose amount of 50, 100, 200 or 400 mg;
even more preferably with a daily dose amount of 200 or 400 mg;
calculated based on the weight of the compound of Formula (I) as the free base;
and preferably comprising
• administering the daily dose amounts by single daily doses, and/or
• administering the composition by intermittent administration of same or different daily doses of the compound of Formula (I), or its isotopic variants, pharmaceutically acceptable salts, co-crystals, hydrates or solvates, in repeating equal or varying intervals of between 4 to 21 days, and/or
• administering the composition over a total treatment period of between 14 to 21 days with administration of the compound with a dose strength of 200 to 600 mg as a single initial loading dose on the first administration day, followed by administration of the composition with a dose strength of 25-100 mg per daily dose on the following days, and/or
• administering the daily dose amounts once only per treatment of an episode.

13. The pharmaceutical composition for the use according to claims 10 to 12, comprising the parenteral administration, preferably intravenous administration or infusion, of the composition to achieve a target minimum concentration of the compound of the Formula (I)
• in the spinal cord of ≥ 1.0 µM, preferably ≥ 5.0 µM, more preferably ≥ 7.0 µM, more preferably ≥ 9.0 µM, even more preferably ≥ 10 µM; and/or
• in the brain of ≥ 500 nM, preferably ≥ 1.0 µM, more preferably ≥ 5.0 µM, more preferably ≥ 7.0 µM, even more preferably ≥ 9.0 µM; and/or
• in the trigeminal ganglia of ≥ 1.0 µM, preferably ≥ 5.0 µM, more preferably ≥ 7.0 µM, more preferably ≥ 9.0 µM, even more preferably ≥ 10 µM; and/or
• in the sacral ganglia of ≥ 2.0 µM, preferably ≥ 5.0 µM, more preferably ≥ 7.0 µM, even more preferably ≥ 9.0 µM; and/or
• in the cerebrospinal fluid (CSF) of ≥ 0.05 µM, preferably ≥ 0.07 µM, more preferably ≥ 0.09 µM; and/or
• in plasma or blood, preferably plasma, of at least 500 nM, preferably at least 1 µM and more preferably at least 2 µM; and/or
to maintain a target minimum plasma concentration
• of at least 500 nM for at least 6 hours, preferably of at least 1 µM for at least 24 hours, preferably for at least 5 days, more preferably for at least 8 days, more preferably for at least 10 days, more preferably for at least 14 days; and/or
• for at least 24 hours up to 5 days, preferably for at least 24 hours up to 8 days, more preferably for at least 24 hours up to 10 days, more preferably for at least 24 hours up to 14 days; and/or
• to achieve an essentially steady stable plasma or blood, preferably plasma, concentration level of the compound of Formula (I) around about 500 nM, preferably about 1 µM and more preferably about 2 µM; and/or
• to achieve an apparent clearance rate (CL/F) of equal to or less than about 2.5 L/h, preferably equal to or less than 1 L/h more preferably less than 0.75 L/h.

14. The pharmaceutical composition according to claims 1 to 9 for the use according to claims 10 to 13 in a combination therapy comprising co-administration of the compound of Formula (**I**), or its isotopic variants, pharmaceutically acceptable salts, co-crystals, hydrates or solvates, with one or more additional pharmaceutically active compounds, preferably one or more antiviral compounds, more preferably Acyclovir,
wherein the co-administration of the combination therapy is carried out in a fixed dose combination therapy by co-administration of the compound of the Formula (I) and the one or more additional pharmaceutically active compounds in a fixed-dose formulation or
wherein the co-administration of the combination therapy is carried out in a free dose combination therapy by co-administration of the compound of the Formula (I) and the one or more additional pharmaceutically active compounds in free doses of the respective compounds, either by simultaneous administration of the individual compounds or by sequential administration of the individual compounds over a time period.

15. A process for the preparation of the pharmaceutical composition according to any one of the claims 1 to 9, comprising the following steps:
(a) preparing a solution of cyclodextrin in water to obtain an aqueous cyclodextrin-solution and optionally adjusting the pH to an acidic pH of < 5.0, preferably pH ≤ 4.0, more preferably pH ≤ 3.0, even more preferably pH ≤ 2.0,
(b) adding and solubilizing the compound of the Formula (I), or its isotopic variant, pharmaceutically acceptable salt, co-crystal, hydrate or solvate, in the cyclodextrin-solution of step (a) so that the resulting solution has an acidic pH of < 5.0, preferably pH ≤ 4.0, more preferably pH ≤ 3.0, even more preferably pH ≤ 2.0,
(c) subsequently adding a pH adjusting agent preferably a buffer, more preferably Na₂HPO₄ or a hydrate thereof such as Na₂HPO₄•12 H₂O, to the solution of step (b) and increasing the pH of the solution to a pH of 6.0 to 7.0, preferably 6.1 to 6.9, more preferably 6.2 to 6.8, more preferably 6.3 to 6.7, most preferred 6.5,
(d) freeze-drying of the solution of step (c) to obtain a lyophilizate;
(e) optionally adding a pharmaceutically acceptable aqueous solution, preferably a physiological solution, more preferably an intravenous injection solution or an infusion solution to the lyophilizate of step (d) to obtain an injectable parenteral pharmaceutical composition;
and wherein optionally in one or more of the steps (a) to (c) and (e) one or more pharmaceutically acceptable carrier and/or one or more excipients, and/or one or more additional active substances being effective in treating a disease or disorder associated with viral infections (antiviral active compounds) are added.
